# EUROPEAN PATENT APPLICATION

(11) **EP 4 470 534 A1**
(43) Date of publication of application: **04.12.2024**
(21) Application number: 23747332.7
(22) Date of filing: 26.01.2023
(51) Int. Cl.: A61K 31/351, A61K 31/166, A61K 31/341, A61K 31/337, A61K 45/06, A61P 25/18, A61P 25/30, A61P 25/24, A61P 25/22

(54) **PHARMACEUTICAL COMPOSITION FOR PREVENTING OR TREATING MENTAL DISORDER**

(30) Priority: 27.01.2022 KR 20220012305
(71) Applicant: Vivozon, Inc., Yongin-si Gyeonggi-do 16914 (KR)
(72) Inventor: CHOI, Dae Kyu, Yongin-si Gyeonggi-do 16914 (KR); YI, Han Ju, Yongin-si Gyeonggi-do 16914 (KR); LEE, Han Mi, Yongin-si Gyeonggi-do 16914 (KR); HEO, Hyun Jin, Yongin-si Gyeonggi-do 16914 (KR); SHIN, Hae Young, Yongin-si Gyeonggi-do 16914 (KR); BAE, Mi Seon, Yongin-si Gyeonggi-do 16914 (KR); KIM, Hyo Jin, Yongin-si Gyeonggi-do 16914 (KR)
(74) Representative: Vossius & Partner Patentanwälte Rechtsanwälte mbB
(86) International application number: PCT/KR2023/001175
(87) International publication number: WO 2023/146291

(57) **Abstract**

Disclosed is a pharmaceutical composition for preventing or treating a mental disorder. More specifically, disclosed is a pharmaceutical composition for treating or preventing a mental disorder, including substance-related and addictive disorder, depressive disorder, anxiety disorder, or post-traumatic stress disorder, by using a compound that simultaneously regulates mGluR5 and 5-HT_{2A}R.

## Description

### TECHNICAL FIELD

Disclosed is a pharmaceutical composition for the prevention or treatment of mental disorder. More specifically, disclosed is a pharmaceutical composition for the prevention or treatment of mental disorder including substance-related and addictive disorder, depressive disorder, anxiety disorder, or post-traumatic stress disorder, by using a compound which modulates mGluR5 and 5-HT_{2A}R at the same time.

### BACKGROUND ART

Mental disorder (psychiatric disorder) is a disorder that causes difficulties in social and occupational adaptation, including interactions with others, due to abnormal symptoms of brain function, and displays various types of behavioral and mental abnormalities. Mental disorder is caused by a complex combination of various causes, including genetic, psychological, biological and environmental factors, but many conditions have unknown causes. When the cause is known, mental disorder usually occurs under severe stressful situations. However, mental disorder experts believe that the main cause is an imbalance in neurotransmitter secretion in the brain-that is, abnormalities in neurotransmitter regulation such as excessive secretion or deficiency. Actually, similar to neurological diseases such as Alzheimer's disease, functional changes have been shown in brain imaging such as magnetic resonance imaging (MRI) and positron emission tomography (PET) (Videbech, Acta Psychiatr Scand., 2000; Fu et al., Exp Ther Med., 2018).

Mental disorders are mainly classified according to the standard of the DSM (Diagnostic and Statistical Manual of Mental Disorders) published by the American Psychiatric Association. DSM-5, currently the 5th edition (2013) of DSM, is used by most mental health specialists. Representative mental disorders classified according to DSM-5 include substance-related and addictive disorders, depressive disorders, anxiety disorders, and trauma- and stressor-related disorders (DSM-5^{™}, 2013).

Substance-related and addictive disorders are broadly classified into substance use disorders and substance-induced disorders. Substances that cause disorders are divided into 10 categories: alcohol, anxiolytics and sedatives, caffeine, cannabis, hallucinogens, inhalants, opioids, stimulants, tobacco and others (including non-substances), all of which directly or indirectly affect brain reward system. By activating the system, they induce craving for substances through pleasure and satisfaction. Substance use disorder is a disorder that involves cognitive, behavioral, and physiological patterns that lead people to continue using substances despite problems arising from substance use, and is distinct from addiction. Substance-induced disorder includes intoxication, withdrawal, and other substance/medication-induced mental disorders. Intoxication is a disorder that causes sensory and emotional problems due to substance use, such as showing severe aggression or hallucinating in an unstable emotional state, putting people in situations where it is difficult to make rational judgments. Withdrawal is a physical and psychological abnormality that occurs when people stop using a substance, and in this case, it also becomes difficult to carry out daily life properly. Additionally, substance-induced mental disorder means that psychotic symptoms are much more severe than those seen in addiction.

Depressive disorder is a mental disorder in which depressive symptoms due to decreased motivation and activities appear continuously and repeatedly, causing various cognitive and psychosomatic disorders, thereby leading to a decline in daily functioning. Symptoms include a significant decrease in motivation due to a depressed mood and despair, which leads to excessive eating or fasting, poor sleep quality or lack of sleep, decreased physical activities, decreased libido, increased fatigue, decreased concentration and the like. Due to feelings of excessive guilt and hopelessness about the future, thoughts about dying or suicide increase, causing great obstacles in social life in many ways. In most cases, long-term treatment is required because the likelihood of recurrence is high.

Anxiety disorder is a general term for mental disorders characterized by various forms of abnormal and pathological fear, worry, anxiety and panic. It is generalized as an overreaction to an ordinary situation with no realistic risk factors, and has an unclear object. Anxiety disorder is characterized by excessive fear, anxiety or avoidance behavior in general situations, and the object or situation is often unclear, and hyperactivities of the autonomic nervous system such as palpitations, increased blood pressure, tachycardia, tremors, dilated pupils, tremors, gastrointestinal disorders and frequent urination are accompanied as physical symptoms. In DSM-IV, anxiety disorders are classified as panic disorder, generalized anxiety disorder, obsessive-compulsive disorder, phobia, post-traumatic stress disorder (PTSD) and acute stress disorder. Post-traumatic stress disorder is a condition in which anxiety persists after experiencing a traumatic event such as war, accident, natural disaster, or severe external mental/physical violence. However, DSM-5 classifies obsessive-compulsive and related disorders, and trauma- and stressor-related disorders (including PTSD) into separate categories.

### DISCLOSURE OF INVENTION

### TECHNICAL PROBLEM

An object of the present invention is to provide a pharmaceutical composition for the prevention or treatment of mental disorder.

Another object of the present invention is to provide a pharmaceutical composition for the prevention or treatment of substance-related and addictive disorder.

Still another object of the present invention is to provide a pharmaceutical composition for the prevention or treatment of depressive disorder, anxiety disorder or post-traumatic stress disorder.

Still another object of the present invention is to provide a method for preventing or treating mental disorder.

Still another object of the present invention is to provide a method for preventing or treating substance-related and addictive disorder.

Still another object of the present invention is to provide a method for preventing or treating depressive disorder, anxiety disorder or post-traumatic stress disorder.

### SOLUTION TO PROBLEM

To achieve the above object, there is provided a pharmaceutical composition for the prevention or treatment of mental disorder comprising a compound of the following Formula 1 or a pharmaceutically acceptable salt thereof, together with a pharmaceutically acceptable carrier or excipient: 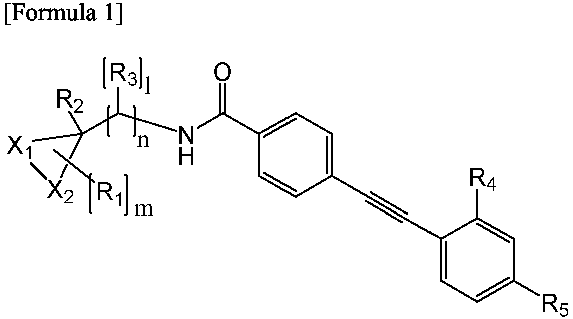 in Formula 1, X₁, X₂, R₁, R₂, R₃, R₄, R₅, 1, m and n are the same as defined herein.

### EFFECTS OF INVENTION

According to the disclosed pharmaceutical composition, mental disorders including substance-related and addictive disorder, depressive disorder, anxiety disorder or post-traumatic stress disorder can be efficiently prevented or treated.

### BRIEF DESCRIPTION OF DRAWINGS

Figures 1 to 3 are graphs showing the results of measuring the drug addiction treatment effects of Test Compounds A, B and C in animal models, respectively.
Figures 4 and 5 are graphs showing the results of measuring the drug addiction prevention effects of Test Compounds A and C in animal models, respectively.
Figures 6 and 7 are graphs showing the results of measuring the effects of Test Compound A on addiction relapse prevention in an animal model, and Figure 8 is a graph showing the results of measuring the effect of Test Compound B on addiction relapse prevention in an animal model.
Figure 9 is a graph showing the results of measuring the withdrawal symptom treatment effect of Test Compound A in an animal model.
Figure 10 is a graph showing the results of measuring the effect of Test Compound A on treating depressive disorder in an animal model.
Figure 11 is a graph showing the results of measuring the effect of Test Compound A on treating anxiety disorder in an animal model.
Figure 12 is a graph showing the results of measuring the effect of Test Compound A on treating post-traumatic stress disorder in an animal model.

### MODE FOR THE INVENTION

The present invention is described in detail hereinafter.

According to one aspect of the present invention, there is provided a pharmaceutical composition for the prevention or treatment of mental disorder comprising a compound of the following Formula 1 or a pharmaceutically acceptable salt thereof, together with a pharmaceutically acceptable carrier or excipient: 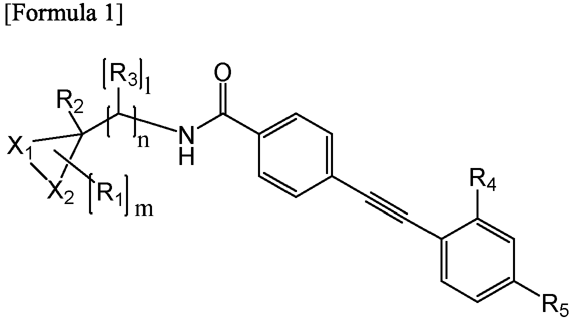 in Formula 1,
X₁ and X₂ together with carbon atom to which they are attached form cyclobutane, cyclopentane, cyclohexane, oxetane, tetrahydrofuran or tetrahydropyran ring;
R₁ is fluoro, hydroxy or C₁-C₅ alkyl;
R₂ is hydrogen, fluoro, hydroxy, C₁-C₅ alkyl or C₁-C₅ alkoxy;
R₃ is hydrogen, deuterium or C₁-C₅ alkyl;
R₄ and R₅ are each independently hydrogen or fluoro, provided that at least one of R₄ and R₅ is fluoro;
l and m are each independently an integer of 0 to 2; and
n is 1 or 2.

According to one embodiment of the present invention, the compound of Formula 1 may be a compound of the following Formula 2: 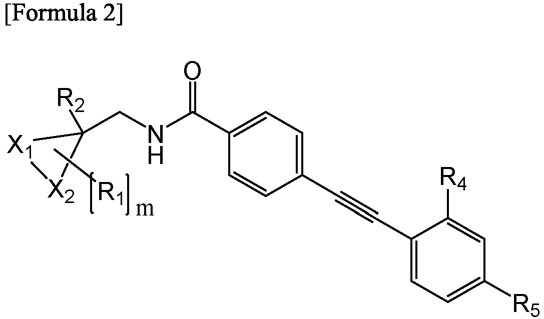 in Formula 2,
X₁ and X₂ together with carbon atom to which they are attached form cyclobutane, cyclopentane, cyclohexane, oxetane, tetrahydrofuran or tetrahydropyran ring;
R₁ is fluoro, hydroxy or methyl;
R₂ is hydrogen, fluoro, hydroxy, methyl, ethyl or methoxy;
R₄ and R₅ are each independently hydrogen or fluoro, provided that at least one of R₄ and R₅ is fluoro; and
m is an integer of 0 to 2.

In another embodiment according to the present invention, representative examples of the compound of Formula 1 may include the following compounds, but are not limited thereto:

**[Table 1]**

| **Compound No.** | **Chemical Structure** | **Chemical Name** |
|---|---|---|
| 1 | 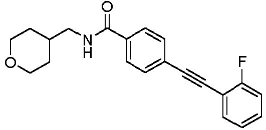 | 4-((2-fluorophenyl)ethynyl)-N-((tetrahydro-2H-pyran-4-yl)methyl)benzamide |
| 2 | 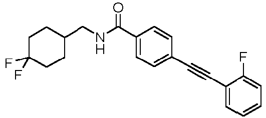 | N-((4,4-difluorocyclohexyl)methyl)-4-((2-fluorophenyl)ethynyl)benzamide |
| 3 | 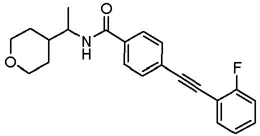 | 4-((2-fluorophenyl)ethynyl)-N-(1-(tetrahydro-2H-pyran-4-yl)ethyl)benzamide |
| 4 | 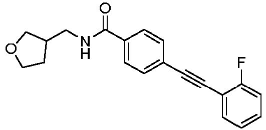 | 4-((2-fluorophenyl)ethynyl)-N-((tetrahydrofuran-3-yl)methyl)benzamide |
| 5 | 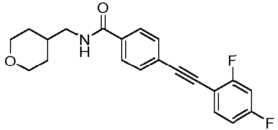 | 4-((2,4-difluorophenyl)ethynyl)-N-((tetrahydro-2H-pyran-4-yl)methyl)benzamide |
| 6 | 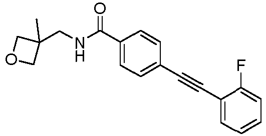 | 4-((2-fluorophenyl)ethynyl)-N-((3-methyloxetan-3-yl)methyl)benzamide |
| 7 | 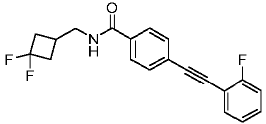 | N-((3,3-difluorocyclobutyl)methyl)-4-((2-fluorophenyl)ethynyl)benzamide |
| 8 | 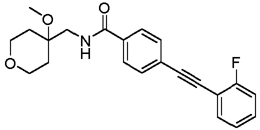 | 4-((2-fluorophenyl)ethynyl)-N-((4-methoxytetrahydro-2H-pyran-4-yl)methyl)benzamide |
| 9 | 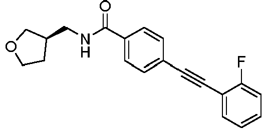 | (R)-4-((2-fluorophenyl)ethynyl)-N-((tetrahydrofuran-3-yl)methyl)benzamide |
| 10 | 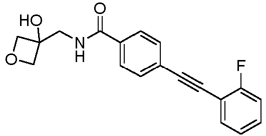 | 4-((2-fluorophenyl)ethynyl)-N-((3-hydroxyoxetan-3-yl)methyl)benzamide |
| 11 | 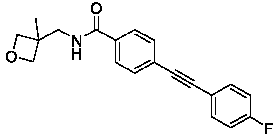 | 4-((4-fluorophenyl)ethynyl)-N-((3-methyloxetan-3-yl)methyl)benzamide |
| 12 | 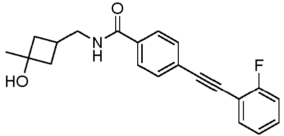 | 4-((2-fluorophenyl)ethynyl)-N-(((1s,3s)-3-hydroxy-3-methylcyclobutyl)methyl)benzamide |
| 13 | 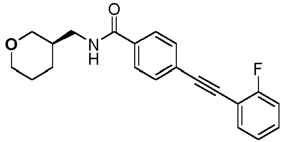 | (R)-4-((2-fluorophenyl)ethynyl)-N-((tetrahydro-2H-pyran-3-yl)methyl)benzamide |
| 14 | 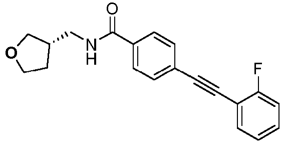 | (S)-4-((2-fluorophenyl)ethynyl)-N-((tetrahydrofuran-3-yl)methyl)benzamide |
| 15 | 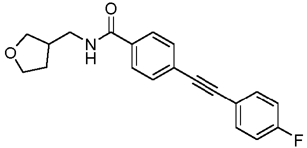 | 4-((4-fluorophenyl)ethynyl)-N-((tetrahydrofuran-3-yl)methyl)benzamide |
| 16 | 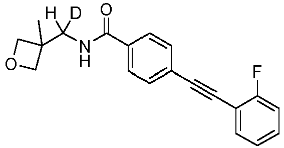 | 4-((2-fluorophenyl)ethynyl)-N-((3-methyloxetan-3-yl)methyl-d)benzamide |
| 17 | 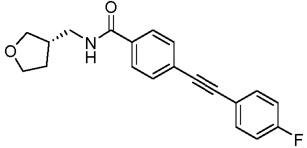 | (S)-4-((4-fluorophenyl)ethynyl)-N-((tetrahydrofuran-3-yl)methyl)benzamide |
| 18 | 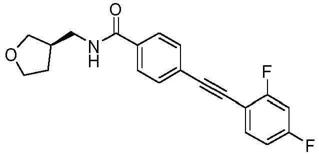 | (R)-4-((2,4-difluorophenyl)ethynyl)-N-((tetrahydrofuran-3-yl)methyl)benzamide |
| 19 | 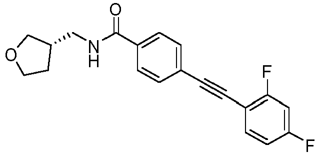 | (S)-4-((2,4-difluorophenyl)ethynyl)-N-((tetrahydrofuran-3-yl)methyl)benzamide |
| 20 | 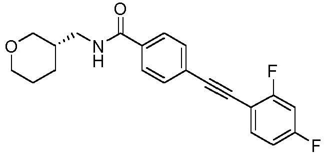 | (S)-4-((2,4-difluorophenyl)ethynyl)-N-((tetrahydro-2H-pyran-3-yl)methyl)benzamide |
| 21 | 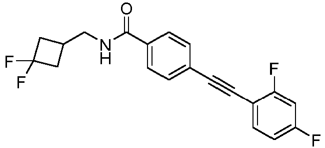 | N-((3,3-difluorocyclobutyl)methyl)-4-((2,4-difluorophenyl)ethynyl)benzamide |
| 22 | 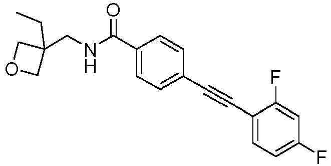 | 4-((2,4-difluorophenyl)ethynyl)-N-((3-ethyloxetan-3-yl)methyl)benzamide |
| 23 | 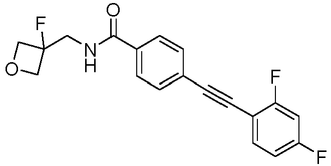 | 4-((2,4-difluorophenyl)ethynyl)-N-((3-fluorooxetan-3-yl)methyl)benzamide |
| 24 | 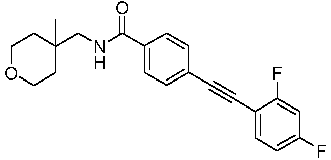 | 4-((2,4-difluorophenyl)ethynyl)-N-((4-methyltetrahydro-2H-pyran-4-yl)methyl)benzamide |
| 25 | 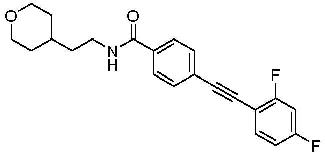 | 4-((2,4-difluorophenyl)ethynyl)-N-(2-(tetrahydro-2H-pyran-4-yl)ethyl)benzamide |
| 26 | 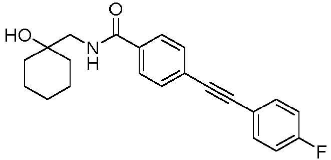 | 4-((4-fluorophenyl)ethynyl)-N-((1-hydroxycyclohexyl)methyl)benzamide |
| 27 | 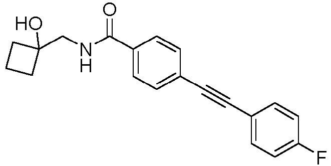 | 4-((4-fluorophenyl)ethynyl)-N-((1-hydroxycyclobutyl)methyl)benzamide |
| 28 | 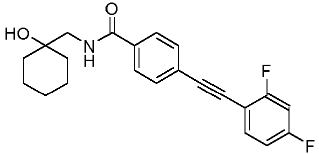 | 4-((2,4-difluorophenyl)ethynyl)-N-((1-hydroxycyclohexyl)methyl)benzamide |
| 29 | 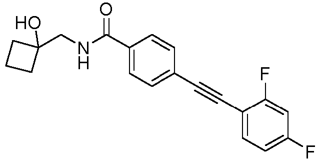 | 4-((2,4-difluorophenyl)ethynyl)-N-((1-hydroxycyclobutyl)methyl)benzamide |
| 30 | 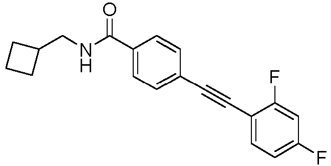 | N-(cyclobutylmethyl)-4-((2,4-difluorophenyl)ethynyl)benzamide |

The compound of Formula 1 acts as a dual modulator of mGluR5 (metabotropic glutamate receptor 5) and 5-HT2A receptor, and has an affinity of 1 µM or less for both receptors.

The compound of Formula 1 according to one embodiment can be prepared by anyone with ordinary knowledge of compound synthesis in this technical field using known compounds or compounds that can be easily prepared therefrom. For example, the compound of Formula 1 can be synthesized according to the methods of Preparation Examples below, but this merely presents one exemplary method and the order of unit operations can be selectively changed as needed. It is not intended to limit the scope of the invention.

### Pharmaceutical Composition

In the present invention, there is provided a pharmaceutical composition for the prevention or treatment of mental disorder comprising a therapeutically effective amount of the compound of Formula 1 or a pharmaceutically acceptable salt thereof, together with a pharmaceutically acceptable carrier or excipient.

The medicinal effect of the compound according to one embodiment of the present invention can be maintained even in the form of a pharmaceutically acceptable salt. The pharmaceutically acceptable salts include both acid or base addition salts and stereochemical isomeric forms thereof. The salt may include any salt that maintains the activity of the parent compound in a subject to be administered and does not cause undesirable effects, and is not specifically limited.

The pharmaceutical composition may be formulated in various oral or parenteral dosage forms. For example, the pharmaceutical composition may be formulated into any dosage form for oral administration, such as tablets, pills, hard/soft capsules, solutions, suspensions, emulsifiers, syrups, granules or elixirs.

When the pharmaceutical composition is formulated into a parenteral dosage form, the pharmaceutical composition may be administered by a parenteral administration method such as subcutaneous injection, intravenous injection, intramuscular injection or intrathoracic injection. The pharmaceutical composition may be prepared as a solution or a suspension by mixing an active ingredient-i.e., the compound of Formula 1 or a pharmaceutically acceptable salt thereof, with a stabilizer or a buffer in water, and the solution or the suspension may be prepared as a unit dosage form of an ampoule or a vial.

The compound of Formula 1 or a pharmaceutically acceptable salt thereof may be comprised in the pharmaceutical composition in an effective dose of 0.1 to 1,000 mg/kg (body weight), preferably 0.5 to 500 mg/kg (body weight) per day for mammals including humans. The pharmaceutical composition may be administered once or divided two or more times a day and administered through an oral or parenteral route.

### Medical Usefulness

According to still another aspect of the present invention, there is provided a method for preventing or treating a mental disorder in a subject, comprising administering to the subject in need thereof a therapeutically effective amount of the compound of Formula 1 or a pharmaceutically acceptable salt thereof. The mental disorder includes substance-related and addictive disorder, depressive disorder, anxiety disorder and post-traumatic stress disorder.

### Substance-Related and Addictive Disorders

One embodiment of the present invention is the provision of a method for preventing or treating substance-related and addictive disorder in a subject, comprising administering to the subject in need thereof a therapeutically effective amount of the compound of Formula 1, or a pharmaceutically acceptable salt thereof.

In another embodiment according to the invention, the substance or drug causing the disorder includes-for example, alcohol; anxiolytics and sedatives (including benzodiazepines, zolpidem, propofol, ketamine, esketamine, phenobarbital, etc.); caffeine; cannabis (including marijuana, synthetic cannabis, etc.); hallucinogens (LSD, phencyclidine, psilocybin, etc.); inhalants (paint thinners, some adhesives, etc.); opioids (fentanyl, morphine, oxycodone, pethidine, methadone, hydromorphone, hydrocodone, oxymorphone, codeine, heroin, etc.); stimulants (methamphetamine, ecstasy, amphetamine, cocaine, etc.); cigarette; and others (including appetite suppressants such as phentermine, phendimetrazine, diethylpropion and mazindol; synthetic anabolic steroids, other commonly abused substances, and non-substances such as gambling, sex, internet, stocks, etc.).

The above ten (10) types of drugs not only have a high potential for causing substance use disorder, but also significantly contribute to the occurrence of substance-induced disorders-that is, the induction of addiction and resulting tolerance by increasing mental/physical dependence due to misuse of drugs, the occurrence of withdrawal symptoms due to reduced use of the drug, and the further development of co-occurring mental disorders associated with the drug itself or the drug treatment process. In addition, the drug may cause a vicious cycle that leads to relapse by failing to suppress the craving for the drug during or upon completion of treatment.
mGluR5 is expressed in the ventral tegmental area (VTA) and the nucleus accumbens of the midbrain, which together form the mesolimbic dopamine pathway, a central neural circuit involved in addiction, so that it is known to play an important role in the addiction process (Lu et al., Molecular Brain Research, 1999; Cleva and Olive, Wiley Interdiscip Rev Membr Transp Signal., 2012).

It has been reported that drug dependence was reduced in mice with the deletion of the mGluR5 gene (Blednov and Harris, Int J Neuropsychopharmacol., 2008). When mGluR5 antagonists were injected into rats addicted to drugs such as cocaine, morphine, amphetamine, ketamine, methamphetamine and nicotine, a decrease in conditioned reward behavior and self-administration and a reduction in symptoms of drug-seeking reinstatement were observed (Cleva and Olive, Wiley Interdiscip Rev Membr Transp Signal., 2012). In addition, the clinical efficacy of mavoglurant, an mGluR5 inhibitory modulator, was confirmed in clinical trials targeting 68 patients with cocaine use disorder. When treated with mGluR5 antagonists, serotonin secretion and subsequent dopamine secretion may be promoted depending on the route of administration and the drug administered. However, as explained below, if dopamine secretion can be prevented by additional 5-HT_{2A}R antagonism, a preventive or curative effect on drug addiction can be expected.

5-HT_{2A}R is expressed in excitatory neurons sending axons to the ventral tegmental area, directly or indirectly affecting the regulation of dopamine, which plays a key role in addiction. An increase of 5-HT_{2A}R has been reported in drug intoxication (Herin, et. al., *Front Psychiatry,* 2013). The 5-HT_{2A}R agonist, 1-[2,5-dimethoxy-4-iodophenyl]-2-aminopropane (DOI) promoted dopamine secretion by increasing the activity of neurons in the ventral tegmental area. It was confirmed that dopamine secretion was inhibited when treated with 5-HT_{2A}R antagonists such as M100907 and ritanserin. In addition, pretreatment with ketanserin, a 5-HT_{2A}R antagonist, suppressed the cocaine-induced increase in dopamine (Bubar and Cunningham, Curr Top Med Chem., 2006), reduced behavioral sensitization symptoms induced by morphine (Gang et. al., 2016), and reduced self-administration restored by signal stimulation in cocaine-addicted rats (Dhonnchadha et. al., Behav Neurosci., 2009).

One embodiment according to the present invention is based on the discovery that the compound of Formula 1 has new and unique pharmacological properties. It has been confirmed that the compound of Formula 1-which has dual modulatory actions on mGluR5 and 5-HT_{2A}R-has the abilities for the prevention and treatment of addiction to abused substances, the treatment of withdrawal symptoms, the prevention of relapse, and the treatment of substance-induced mental disorders such as accompanying depression and anxiety in animal models.

For example, patients are at risk of becoming addicted to or become addicted to therapeutic substances administered to treat disorders or diseases. For instance, there may be a risk of addiction when exposed to addictive therapeutic substances such as narcotic opioid analgesics. Given this situation, the compound of Formula 1 may be provided to patients alone or in combination with other additional therapeutic agents, together with an addictive therapeutic substance. The compound of Formula 1 may be provided for the prevention or treatment of addiction secondary to opioid analgesics administered to patients suffering from pain or at risk of pain.

The compound of Formula 1 may be used in combination with an anti-addiction agent including a drug of substitution or a drug of replacement. The anti-addiction agent may be naloxone, naltrexone, nalmefene, disulfiram, acamprosate, topiramate, risperidone, paliperidone, ondansetron, fluoxetine, sertraline, paroxetine, citalopram, fluvoxamine, venlafaxine, duloxetine, mirtazapine or bupropion. For example, naloxone can be administered as an antidote to opioids, and in this case, the compound of Formula 1 can treat withdrawal symptoms induced by naloxone. In this way, the compound of Formula 1 can be provided to control withdrawal symptoms and accompanying mental disorders.

In addition, the compounds of Formula 1 may be provided for preventing recurrence or reducing the likelihood of recurrence of abuse of the substance inducing the substance-related and addictive disorder in a patient attempting to discontinue use of the substance inducing the substance-related and addictive disorder.

### Depressive Disorder

Another embodiment of the present invention is the provision of a method for preventing or treating depressive disorder in a subject, comprising administering to the subject in need thereof a therapeutically effective amount of the compound of Formula 1, or a pharmaceutically acceptable salt thereof.

Selective serotonin reuptake inhibitor (SSRI) drugs-which are widely used for the treatment of depressive disorder-not only have various side effects such as vomiting, diarrhea, headache, dizziness, insomnia, anxiety, loss of appetite and libido, and suicidal impulses, but also generally take several weeks to show therapeutic effects. In addition, about one-third of patients prescribed SSRIs do not exhibit antidepressant efficacy. It has been known that ketamine-which is an antagonist against N-methyl-D-aspartate (NMDA) receptor (NMDAR)-is clinically effective for patients with treatment-resistant depression.

Because mGluR5 antagonists partially inhibit NMDAR function, their potential as a therapeutic agent for depressive disorder is attracting attention (Barnes et al., Biol Psychiatry, 2018). In the mouse tail suspension test which is an animal model related to depressive disorder, MPEP-which is an mGluR5 antagonist-decreased immobility time in a dose-dependent manner. Additionally, in the rat forced swim test, MTEP (3-[(2-methyl-1,3-thiazol-4-yl)ethynyl]pyridine)-which is another mGluR5 antagonist-demonstrated the effect of reducing immobility time (Tatarczynska et al., Br J Pharmacol., 2001). Indirect inhibition of NMDAR through mGluR5 antagonists will effectively treat depressive disorder by reducing symptoms such as increased arterial pressure/heart rate/cardiac output, hallucinations, delirium and cognitive disorder, which are side effects of direct NMDAR antagonists such as ketamine.

5-HT_{2A}R antagonists are known to be effective in improving treatment-resistant depression when used together with SSRIs. In practice, mirtazapine and mianserin are clinically used as adjunctive therapeutic agents for treatment-resistant depression (Marek et al., Neuropsychopharmacology, 2003; Celada et al., Rev Psychiatr Neurosci., 2004).

Therefore, the compound of Formula 1-which has dual modulatory actions on mGluR5 and 5-HT_{2A}R-can be used for the prevention and treatment of depressive disorder, specifically treatment-resistant depression.

### Anxiety Disorder and Post-Traumatic Stress Disorder

Another embodiment of the present invention is the provision of a method for preventing or treating anxiety disorder and post-traumatic stress disorder (PTSD) in a subject, comprising administering to the subject in need thereof a therapeutically effective amount of the compound of Formula 1, or a pharmaceutically acceptable salt thereof.

In the amygdala, a representative anxiety-related central region, mGluR5 is known to play an important role in the acquisition of fear and anxiety behavior. It has been shown that treatment with MPEP-an mGluR5 antagonist-inhibits the fear/anxiety acquisition process by inhibiting the formation of long-term potentiation (LTP) in the amygdala. (Rodrigues et. al., J Neurosci., 2002). When MPEP is injected into the amygdala, the time the test animals spend in the bright area increased in the Light-Dark Box test, and the time the test animals spend in the open arm and the number of entries into the open arm increased in the Elevated-Plus Maze test. These results demonstrate remarkable anti-anxiety effects (de ra Mora et al., Eur J Neurosci, 2006).

In addition, because 5-HT_{2A}R in the amygdala is also closely related to anxiety behavior, it has been known that anxiety behavior is accelerated when 5-HT_{2A}R agonist is administered directly to the amygdala. In practice, 5-HT_{2A}R antagonists such as mirtazapine, trazodone and mianserin are clinically used as therapeutic agents for anxiety disorders (Murphy, Br J Clin Pharmac., 1978; Chea and Giorgi, Am JAni Yet Sci., 2017).

Therefore, the compound of Formula 1-which has dual modulatory actions on mGluR5 and 5-HT_{2A}R-can be used for the prevention and treatment of anxiety disorder and post-traumatic stress disorder.

The specific administration method and therapeutically effective amount of the compound of Formula 1 or a pharmaceutically acceptable salt thereof can be readily determined by a person skilled in the art, taking into account the type of target mammal, the type of disorder and the type of the compound of Formula 1 and the like, and there is no specific limitation thereto.

### [Examples]

Hereinafter, the present invention is explained in more detail with the following examples. However, it must be understood that the protection scope of the present disclosure is not limited to the examples.

### Preparation Example 1: Synthesis of 4-((2-fluorophenyl)ethynyl)-N ((tetrahydro-2H pyran-4-yl)methyl)benzamide (Compound 1)

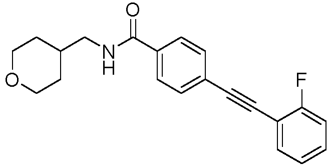

### Step 1: Synthesis of 4-((2-fluorophenyl)ethynyl)benzoic acid

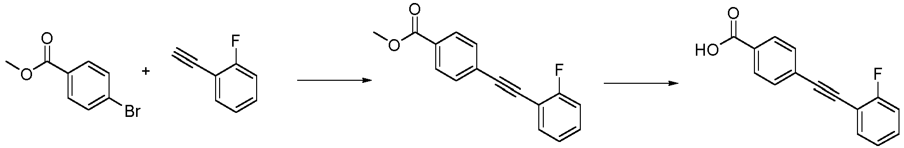

1-Ethynyl-2-fluorobenzene (0.500 g, 4.162 mmol), methyl 4-bromobenzoate (0.940 g, 4.370 mmol), copper iodide (0.016 g, 0.083 mmol) and tetrakis(triphenylphosphine)palladium (0.096 g, 0.083 mmol) and triethylamine (5.801 mL, 41.622 mmol) were dissolved in toluene (10 mL) at room temperature. The obtained solution was stirred at 95°C for 8 hours, and then the temperature was lowered to room temperature to terminate the reaction. The reaction mixture was filtered through a Celite pad to remove solids, and water was added to the filtrate, followed by extraction with dichloromethane. The organic layer was washed with a saturated aqueous sodium chloride solution, dried over anhydrous magnesium sulfate, filtered and concentrated under reduced pressure. The concentrate was purified and concentrated by column chromatography to obtain methyl 4-((2-fluorophenyl)ethynyl)benzoate (0.775 g, 73.2%) as a white solid.

Then, the solution in which the obtained product and lithium hydroxide monohydrate (0.256 g, 6.096 mmol) were dissolved in tetrahydrofuran (5 mL)/methanol (5 mL)/water (5 mL) at room temperature was stirred at 50°C for 6 hours, and then the temperature was lowered to room temperature to terminate the reaction. After removing the solvent from the reaction mixture under reduced pressure, 1N hydrochloric acid aqueous solution was added to the concentrate and stirred. The precipitated solid was filtered, washed with water and dried to obtain 4-((2-fluorophenyl)ethynyl)benzoic acid (0.703 g, 96.0%) as a pale yellow solid: **LRMS** (ES) *m*/*z* 239.12 [M-H]⁺, calculated MW 240.23.

### Step 2: Synthesis of 4-((2-fluorophenyl)ethynyl)-H-((tetrahydro-2H-pyran-4-yl)methyl)benzamide

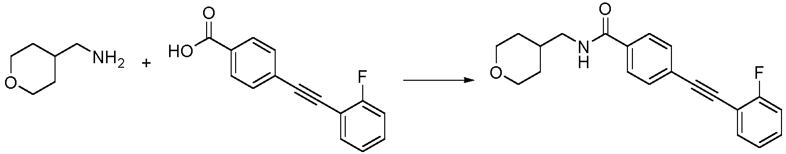

4-((2-Fluorophenyl)ethynyl)benzoic acid (0.070 g, 0.291 mmol) and (tetrahydro-2H-pyran-4-yl)methanamine (0.050 g, 0.437 mmol), 1-ethyl-3-(3-dimethylaminopropyl)carbodiimide hydrochloride (EDC-HCl, 0.084 g, 0.437 mmol), 1*H-*benzo[*d*][1,2,3]triazol-1-ol (HOBt, 0.059 g, 0.437 mmol) and *N,N*-diisopropylethylamine (0.254 mL, 1.457 mmol) were dissolved in *N,N-*dimethylformamide (3 mL) at room temperature and stirred at the same temperature for 18 hours. Saturated aqueous sodium hydrogen carbonate solution was poured into the reaction mixture, followed by extraction with ethyl acetate. The organic layer was washed with saturated aqueous sodium chloride solution, dried over anhydrous magnesium sulfate, filtered and concentrated under reduced pressure. The concentrate was purified and concentrated by column chromatography to obtain 4-((2-fluorophenyl)ethynyl)-*N*-((tetrahydro-2*H*-pyran-4-yl)methyl)benzamide (0.075 g, 76.3%) as a white solid: **LRMS** (ES) *m*/*z* 338.21 [M+H]⁺, calculated MW 337.39; **¹H-NMR** (400 MHz, CD₃OD) d 7.82 (d, *J =* 8.8 Hz, 2 H), 7.60 (d, *J =* 8.8 Hz, 2 H), 7.55 (t, *J =* 7.6 Hz, 1 H), 7.45 ~ 7.37 (m, 1 H), 7.21 ~ 7.14 (m, 2 H), 3.93 (dd, *J =* 11.2, 4.0 Hz, 2 H), 3.39 (t, *J =* 11.8 Hz, 2 H), 3.28 ~ 3.26 (m, 2 H), 1.90 ~ 1.86 (m, 1 H), 1.69 (s, 2 H), 1.37 ~ 1.27 (m, 2 H).

### Preparation Example 2: Synthesis of N-((4,4-difluorocyclohexyl)methyl)-4-((2-fluorophenyl)ethynyl)benzamide (Compound 2)

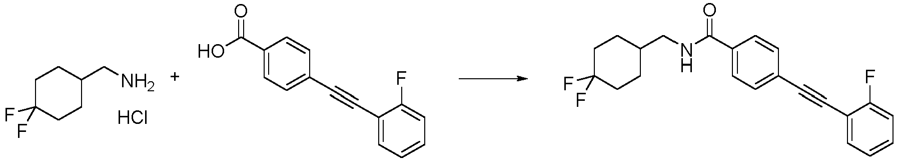

4-((2-Fluorophenyl)ethynyl)benzoic acid (0.200 g, 0.833 mmol) and (4,4-difluorocyclohexyl)methanamine hydrochloride (0.232 g, 1.249 mmol) as starting materials were used in a similar manner to Step 2 of Preparation Example 1 to obtain *N*-((4,4-difluorocyclohexyl)methyl)-4-((2-fluorophenyl)ethynyl)benzamide (0.175 g, 56.6%) as white solid: **LRMS** (ES) m/z 372.23 [M+H]⁺, calculated MW 371.4; **¹H-NMR** (400 MHz, DMSO-*d*₆) d 8.63 (t, *J =* 6.0 Hz, 1 H), 7.87 (d, *J =* 8.8 Hz, 2 H), 7.65 ~ 7.61 (m, 3 H), 7.49 ~ 7.45 (m, 1 H), 7.33 (t, *J =* 9.2 Hz, 1 H), 7.25 (td, *J =* 7.6, 1.2 Hz, 1 H), 3.14 (t, *J =* 6.4 Hz, 2 H), 2.01 ~ 1.96 (m, 2 H), 1.81 ~ 1.66 (m, 5 H), 1.22 ~ 1.13 (m, 2 H).

### Preparation Example 3: Synthesis of 4-((2-fluorophenyl)ethynyl)-N-(1-(tetrahydro-2H pyran-4-yl)ethyl)benzamide (Compound 3)

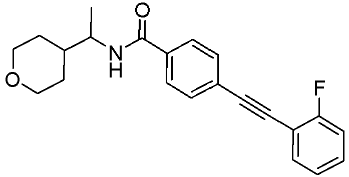

### Step 1: Synthesis of (Z)-2-methyl-N-((tetrahydro-2H-pyran-4-yl)methylene)propane-2-sulfinamide

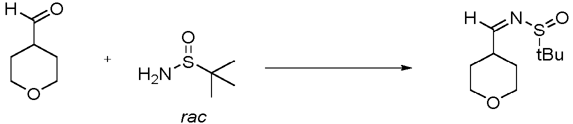

2-Methylpropane-2-sulfinamide (1.000 g, 8.251 mmol), tetrahydro-2*H*-pyran-4-carbaldehyde (2.072 g, 18.152 mmol), magnesium sulfate (MgSO₄, 5.462 g, 45.380 mmol) and pyridinium *p*-toluenesulfonate (0.107 g, 0.495 mmol) were dissolved in 1,2-dichloroethane (14 mL) at room temperature, and the obtained solution was stirred at the same temperature under visible light (Blue LED, 40W) for 24 hours. Water was poured into the reaction mixture, followed by extraction with dichloromethane. The organic layer was washed with a saturated aqueous sodium chloride solution, dried over anhydrous sodium sulfate, filtered and concentrated under reduced pressure. The concentrate was purified by column chromatography and concentrated to obtain (Z)-2-methyl-*N-*((tetrahydro-2*H-*pyran-4-yl)methylene)propane-2-sulfinamide (0.800 g, 44.6%) as white solid.

### Step 2: Synthesis of 1-(tetrahydro-2H-pyran-4-yl)ethan-1-amine hydrochloride

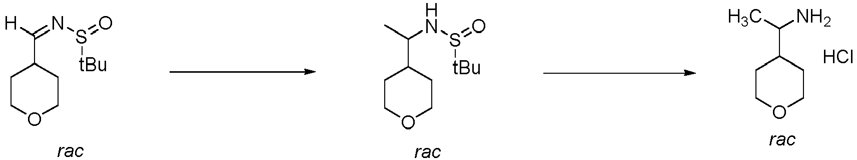

(Z)-2-methyl-*N-*((tetrahydro-2*H-*pyran-4-yl)methylene)propane-2-sulfinamide (0.800 g, 3.681 mmol) and methylmagnesium bromide (1.40 M solution in THF/toluene, 5.259 mL, 7.362 mmol) were dissolved in dichloromethane (15 mL), and the resulting solution was stirred at room temperature for 1 hour and further stirred at the same temperature for 18 hours. Water was poured into the reaction mixture, followed by extraction with dichloromethane. The organic layer was washed with a saturated aqueous sodium chloride solution, dried over anhydrous sodium sulfate, filtered and concentrated under reduced pressure. The concentrate was purified by column chromatography and concentrated to obtain 2-methyl-*N*-(1-(tetrahydro-2*H*-pyran-4-yl)ethyl)propane-2-sulfinamide (0.475 g, 55.3 %) as colorless liquid. 0.400 g (1.714 mmol) of obtained product and hydrogen chloride (4.00 M solution in 1,4-dioxane, 1.286 mL, 5.142 mmol) were dissolved in methanol (2 mL) at room temperature, and the resulting solution was stirred at the same temperature for 30 minutes. After removing the solvent from the reaction mixture under reduced pressure, ethyl acetate (1 mL) and hexane (10 mL) were added to the concentrate and stirred. The precipitated solid was filtered, washed with hexane and dried to obtain 1-(tetrahydro-2*H*-pyran-4-yl)ethan-1-amine hydrochloride (0.143 g, 50.4%) as pink solid.

### Step 3: Synthesis of 4-((2-fluorophenyl)ethynyl)-N-(1-(tetrahydro-2H-pyran-4-yl)ethyl)benzamide

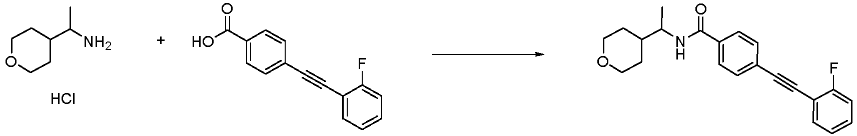

1-(Tetrahydro-2H-pyran-4-yl)ethan-1-amine hydrochloride (0.035 g, 0.211 mmol), 4-((2-fluorophenyl)ethynyl)benzoic acid (0.051 g, 0.211 mmol), 1-[bis(dimethylamino)methylene]-1H-1,2,3-triazolo[4,5-b]pyridinium 3-oxide hexafluorophosphate (HATU, 0.104 g, 0.275 mmol) and N,N-diisopropylethylamine (0.184 mL, 1.056 mmol) were dissolved in N,N-dimethylformamide (2 mL) at room temperature, and the obtained solution was stirred at the same temperature for 5 hours. The solvent was removed from the reaction mixture under reduced pressure, and a saturated aqueous sodium hydrogen carbonate solution was poured into the obtained concentrate, followed by extraction with ethyl acetate. The organic layer was washed with saturated aqueous sodium chloride solution, dried over anhydrous magnesium sulfate, filtered and concentrated under reduced pressure. The concentrate was purified and concentrated by column chromatography to obtain 4-((2-fluorophenyl)ethynyl)-*N-*(1-(tetrahydro-2*H-*pyran-4-yl)ethyl)benzamide (0.045 g, 60.6%) was obtained as white solid: **LRMS** (ES) *m*/*z* 352.13 [M+H]⁺, calculated MW 351.42; **¹H-NMR** (400 MHz, DMSO-*d*₆) d 8.27 (d, *J =* 8.4 Hz, 1 H), 7.87 (d, *J =* 8.4 Hz, 2 H), 7.63 ~ 7.61 (m, 3 H), 7.46 (m, 1 H), 7.32 (t, *J =* 15.4 Hz, 1 H), 7.26 (t, *J =* 4.0 Hz, 1 H), 3.88 ~ 3.77 (m, 3 H), 3.24 (q, *J =* 14.0 Hz, 2 H), 1.62 ~ 1.56 (m, 3 H), 1.22 ~ 1.15 (m, 2 H), 1.09 (d, *J* = 6.8 Hz, 3 H).

### Preparation Example 4: Synthesis of 4-((2-fluorophenyl)ethynyl)-N-((tetrahydrofuran-3-yl)methyl)benzamide (Compound 4)

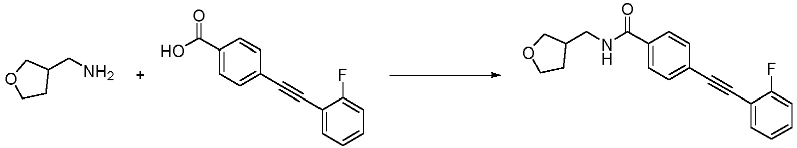

4-((2-Fluorophenyl)ethynyl)benzoic acid (0.150 g, 0.624 mmol) and (tetrahydrofuran-3-yl)methanamine (0.098 mL, 0.937 mmol) as starting materials were used in a similar manner to Step 2 of Preparation Example 1 to obtain 4-((2-fluorophenyl)ethynyl)-*N-*((tetrahydrofuran-3-yl)methyl)benzamide (0.143 g, 70.8%) as white solid: **LRMS** (ES) *m*/*z* 324.21 [M+H]⁺, calculated MW 323.37; **¹H-NMR** (400 MHz, DMSO-*d*₆) d 8.68 (t, *J =* 5.6 Hz, 1 H), 7.86 (dd, *J* = 6.8, 2.0 Hz, 2 H), 7.64 ~ 7.59 (m, 3 H), 7.50 ~ 7.44 (m, 1 H), 7.33 (t, *J =* 8.6 Hz, 1 H), 7.25 (td, *J =* 7.6, 0.9 Hz, 1 H), 3.71 ~ 3.54 (m, 3 H), 3.45 ~ 3.36 (m, 2 H), 3.30 ~ 3.13 (m, 2 H), 1.93 ~ 1.87 (m, 1 H), 1.62 ~ 1.50 (m, 1 H).

### Preparation Example 5: Synthesis of 4-((2,4-difluorophenyl)ethynyl)-N-((tetrahydro-2H-pyran-4-yl)methyl)benzamide (Compound 5)

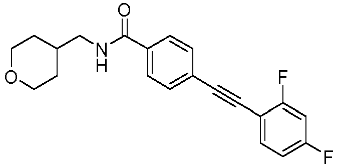

### Step 1: Synthesis of 4-((2,4-difluorophenyl)ethynyl)benzoic acid

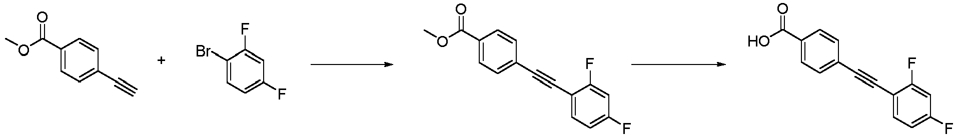

Methyl 4-ethynylbenzoate (2.000 g, 12.487 mmol) and 1-bromo-2,4-difluorobenzene (2.410 g, 12.487 mmol) were used in a similar manner to Step 1 of Preparation Example 1 to obtain methyl 4-((2,4-difluorophenyl)ethynyl)benzoate (0.780 g, 22.9%) as white solid, followed by obtaining 4-((2,4-difluorophenyl)ethynyl)benzoic acid (0.730 g, 98.7%) as white solid: **LRMS** (ES) *m*/*z* 256.98 (*M*-1) [M+H]⁺, calculated MW 258.22.

### Step 2: Synthesis of 4-((2,4-difluorophenyl)ethynyl)-N-((tetrahydro-2H-pyran-4-yl)methyl)benzamide

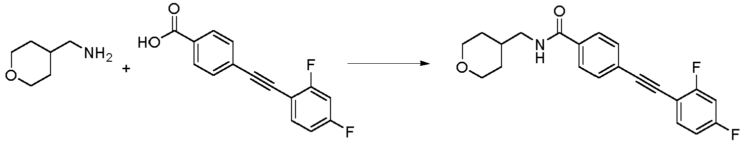

4-((2,4-Difluorophenyl)ethynyl)benzoic acid (0.150 g, 0.581 mmol) and (tetrahydro-2*H-*pyran-4-yl)methanamine (0.074 g, 0.639 mmol) were used in a similar manner to Step 3 of Preparation Example 3 to obtain 4-((2,4-difluorophenyl)ethynyl)-*N*-((tetrahydro-2*H*-pyran-4-yl)methyl)benzamide (0.156 g, 75.6%) as white solid: **LRMS** (ES) *m*/*z* 356.42 [M+H]⁺, calculated MW 355.38; **¹H-NMR** (400 MHz, DMSO-*d*₆) d 8.58 (t, *J =* 5.6 Hz, 1 H), 7.86 (d, *J =* 8.4 Hz, 2 H), 7.70 (q, *J =* 7.9 Hz, 1 H), 7.61 (d, *J =* 8.0 Hz, 2 H), 7.42 (td, *J =* 9.6, 2.8 Hz, 1 H), 7.17 (td, *J* = 7.8, 2.5 Hz, 1 H), 3.80 (dd, *J =* 11.2, 2.4 Hz, 2 H), 3.22 (t, *J =* 11.8 Hz, 2 H), 3.12 (t, *J =* 6.2 Hz, 2 H), 1.78 ~ 1.73 (m, 1 H), 1.55 (d, *J =* 12.8 Hz, 2 H), 1.20 ~ 1.10 (m, 2 H).

### Preparation Example 6: Synthesis of 4-((2-fluorophenyl)ethynyl)-N-((3-methyloxetan-3-yl)methyl)benzamide (Compound 6)

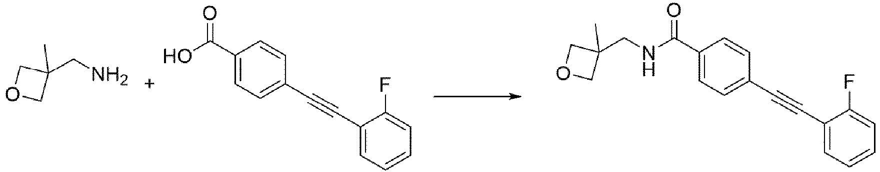

4-((2-Fluorophenyl)ethynyl)benzoic acid (0.070 g, 0.291 mmol) and (3-methyloxetan-3-yl)methanamine (0.044 g, 0.437 mmol) as starting materials were used in a similar manner to Step 2 of Preraration Example 1 to obtain 4-((2-fluorophenyl)ethynyl)-*N*-((3-methyloxetan-3-yl)methyl)benzamide (0.052 g, 55.2%) as white solid: **LRMS** (ES) *m*/*z* 324.33 [M+H]⁺, calculated MW 323.37; **¹H-NMR** (400 MHz, DMSO-*d*₆) d 8.71 (t, J = 6.0 Hz, 1 H), 7.88 (dd, J = 6.4, 2.0 Hz, 2 H), 7.66 ~ 7.61 (m, 3 H), 7.50 ~ 7.45 (m, 1 H), 7.33 (td, J = 9.1, 1.1 Hz, 1 H), 7.25 (td, J = 7.6, 1.1 Hz, 1 H), 4.44 (d, J = 5.2 Hz, 2 H), 4.17 (d, J = 5.2 Hz, 2 H), 3.44 (d, J = 6.0 Hz, 2 H), 1.22 (s, 3 H).

### Preparation Example 7: Synthesis of N-((3,3-difluorocyclobutyl)methyl)-4-((2-fluorophenyl)ethynyl)benzamide (Compound 7)

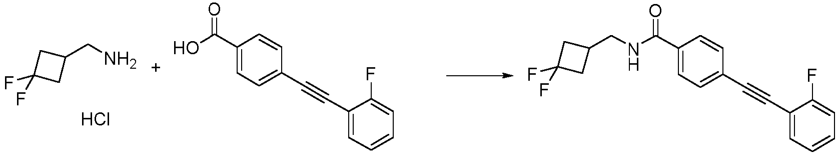

4-((2-Fluorophenyl)ethynyl)benzoic acid (0.070 g, 0.291 mmol) and (3,3-difluorocyclobutyl)methanamine hydrochloride (0.069 g, 0.437 mmol) as starting materials were used in a similar manner to Step 3 of Preparation Example 3 to obtain *N-*((3,3-difluorocyclobutyl)methyl)-4-((2-fluorophenyl)ethynyl)benzamide (0.075 g, 75.0%) as white solid: **LRMS** (ES) *m*/*z* 344.22 [M+H]⁺, calculated MW 343.35; **¹H-NMR** (400 MHz, DMSO-*d*₆) d 8.72 (t, *J =* 5.6 Hz, 1 H), 7.86 (d, *J =* 7.6 Hz, 2 H), 7.64 ~ 7.61 (m, 3 H), 7.47 (q, *J =* 6.4 Hz, 1 H), 7.33 (t, *J =* 9.2 Hz, 1 H), 7.25 (t, *J =* 7.6 Hz, 1 H), 2.63 ~ 2.55 (m, 3 H), 2.38 ~ 2.29 (m, 4 H).

### Preparation Example 8: Synthesis of 4-((2-fluorophenyl)ethynyl)-N ((4-methoxytetrahydro-2H-pyran-4-yl)methyl)benzamide (Compound 8)

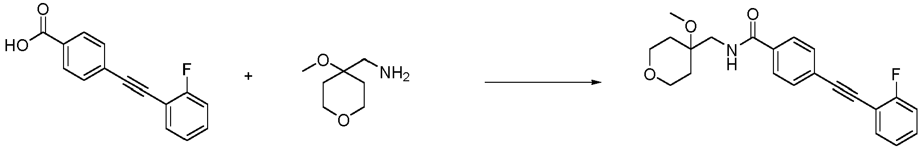

4-((2-Fluorophenyl)ethynyl)benzoic acid (0.070 g, 0.291 mmol) and (4-methoxytetrahydro-2*H-*pyran-4-yl)methanamine (0.042 g, 0.291 mmol) as starting materials were used in a similar manner to Step 3 of Preparation Example 3 to obtain 4-((2-fluorophenyl)ethynyl)-*N*-((4-methoxytetrahydro-2*H*-pyran-4-yl)methyl)benzamide (0.080 g, 74.7%) as pale yellow oil: **LRMS** (ES) *m*/*z* 368.20 [M+H]⁺, calculated MW 367.42; **¹H-NMR** (400 MHz, CDCl₃) d 7.76 (d, *J =* 8.0 Hz, 2 H), 7.61 (d, *J =* 7.6 Hz, 2 H), 7.52 (m, 1 H), 7.35 ~ 7.33 (m, 1 H), 7.16 ~ 7.11 (m, 2 H), 6.32 (bs, 1 H), 3.73 (m, 4 H), 3.57 (d, *J =* 4.8 Hz, 2 H), 3.26 (s, 3 H), 1.82 ~ 1.67 (m, 4 H).

### Preparation Example 9: Synthesis of (R)-4-((2-fluorophenyl)ethynyl)-N ((tetrahydrofuran-3-yl)methyl)benzamide (Compound 9)

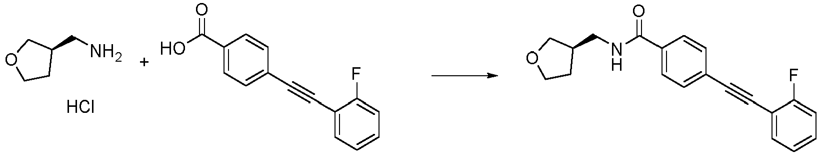

4-((2-Fluorophenyl)ethynyl)benzoic acid (0.070 g, 0.291 mmol) and (R)-(tetrahydrofuran-3-yl)methanamine hydrochloride (0.043 g, 0.321 mmol) as starting materials were used in a similar manner to Step 3 of Preparation Example 3 to obtain (R)-4-((2-fluorophenyl)ethynyl)-*N-*((tetrahydrofuran-3-yl)methyl)benzamide (0.080 g, 84.9%) as white solid: **LRMS** (ES) *m*/*z* 324.23 [M+H]⁺, calculated MW 323.37; **¹H-NMR** (400 MHz, DMSO-*d*₆) d 8.67 (t, *J =* 5.4 Hz, 1 H), 7.86 (d, *J =* 8.4 Hz, 2 H), 7.64 ~ 7.62 (m, 3 H), 7.50 ~ 7.45 (m, 1 H), 7.33 (t, *J =* 9.2 Hz, 1 H), 7.25 (t, *J =* 7.6 Hz, 1 H), 3.71 ~ 3.55 (m, 3 H), 3.45 ~ 3.42 (m, 1 H), 3.27 ~ 3.19 (m, 2 H), 3.13 ~ 3.12 (m, 1 H), 1.95 ~ 1.86 (m, 1 H), 1.60 ~ 1.52 (m, 1 H).

### Preparation Example 10: Synthesis of 4-((2-fluorophenyl)ethynyl)-N-((3-hydroxyoxetan-3-yl)methyl)benzamide (Compound 10)

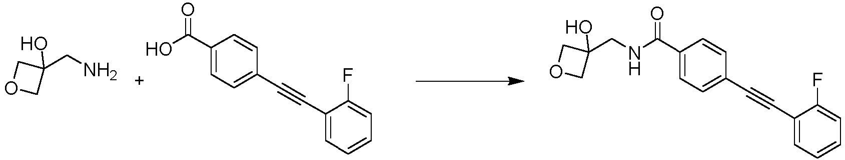

4-((2-Fluorophenyl)ethynyl)benzoic acid (0.100 g, 0.416 mmol) and 3-(aminomethyl)oxetan-3-ol (0.052 g, 0.500 mmol) as starting materials were used in a similar manner to Step 3 of Preparation Example 3 to obtain 4-((2-fluorophenyl)ethynyl)-*N*-((3-hydroxyoxetan-3-yl)methyl)benzamide (0.082 g, 60.5%) as ivory solid: **LRMS** (ES) *m*/*z* 326.22 [M+H]⁺, calculated MW 325.34; **¹H-NMR** (400 MHz, DMSO-*d*₆) d 8.71 (t, *J =* 6.2 Hz, 1 H), 7.90 (dd, *J =* 6.8, 1.6 Hz, 2 H), 7.65 ~ 7.61 (m, 3 H), 7.49 ~ 7.47 (m, 1 H), 7.33 (t, *J =* 8.4 Hz, 1 H), 7.25 (td, *J =* 7.5, 0.9 Hz, 1 H), 5.86 (s, 1 H), 4.46 (d, *J =* 6.8 Hz, 2 H), 4.36 (d, *J =* 6.4 Hz, 2 H), 3.53 (d, *J =* 6.0 Hz, 2 H).

### Preparation Example 11: Synthesis of 4-((4-fluorophenyl)ethynyl)-N-((3-methyloxetan-3-yl)methyl)benzamide (Compound 11)

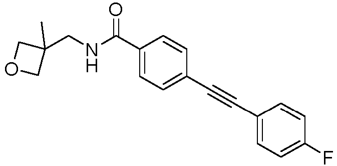

### Step 1: Synthesis of 4-((4-fluorophenyl)ethynyl)benzoic acid

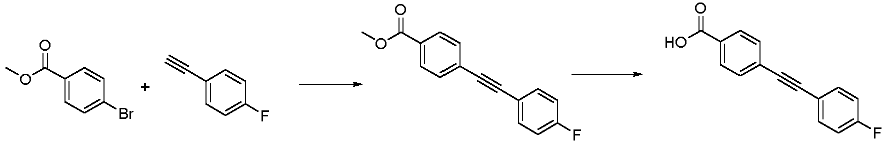

Methyl 4-bromobenzoate (0.985 g, 4.578 mmol) and 1-ethanyl-4-fluorobenzene (0.500 g, 4.162 mmol) as starting materials were used in a similar manner to Step 3 of Preparation Example 3 to obtain methyl 4-((4-fluorophenyl)ethynyl)benzoate (0.779 g, 73.6%) as white solid, followed by obtaining 4-((4-fluorophenyl)ethynyl)benzoic acid (0.672 g). , 91.3%) as light yellow solid: **LRMS** (ES) *m*/*z* 239.05 [M-H]⁺, calculated MW 240.23.

### Step 2: Synthesis of 4-((4-fluorophenyl)ethynyl)-N-((3-methyloxetan-3-yl)methyl)benzamide

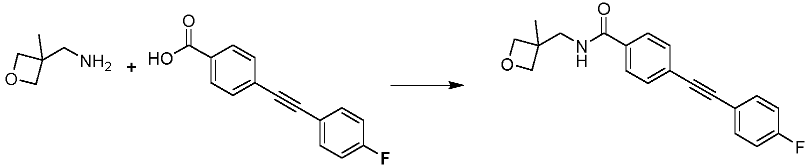

4-((4-Fluorophenyl)ethynyl)benzoic acid (0.070 g, 0.291 mmol) and (3-methyloxetan-3-yl)methanamine (0.035 g, 0.350 mmol) as starting materials were used in a similar manner to Step 3 of Preparation Example 3 to obtain 4-((4-fluorophenyl)ethynyl)-N-((3-methyloxetan-3-yl)methyl)benzamide (0.089 g, 94.5%) as white solid: **LRMS** (ES) *m*/*z* 324.11 [M+H]⁺, calculated MW 323.37; **¹H-NMR** (400 MHz, DMSO-*d*₆) d 8.69 (t, *J =* 6.2 Hz, 1 H), 7.87 (d, *J =* 8.0 Hz, 2 H), 7.64 ~ 7.58 (m, 4 H), 7.29 ~ 7.23 (m, 2 H), 4.44 (d, *J =* 5.6 Hz, 2 H), 4.17 (d, *J =* 6.0 Hz, 2 H), 3.43 (d, *J =* 6.0 Hz, 2 H), 1.22 (s, 3 H).

### Preparation Example 12: Synthesis of 4-((2-fluorophenyl)ethynyl)-N-(((1s,3s)-3-hydroxy-3-methylcyclobutyl)methyl)benzamide (Compound 12)

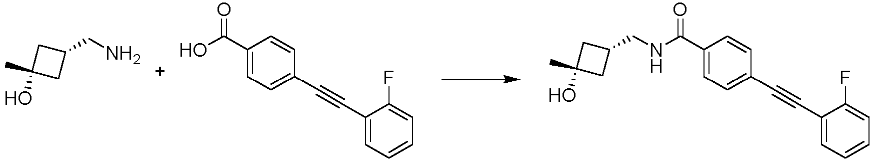

4-((2-Fluorophenyl)ethynyl)benzoic acid (0.070 g, 0.291 mmol) and (1*s*,3*s*)-3-(aminomethyl)-1-methylcyclobutan-1-ol (0.044 g, 0.379 mmol) as starting materials were used in a similar manner to Step 3 of Preparation Example 3 to obtain 4-((2-fluorophenyl)ethynyl)-*N-*(((1*s*,3*s*)-3-hydroxy-3-methylcyclobutyl)methyl)benzamide (0.097 g, 98.7%) as white solid: **LRMS** (ES) *m*/*z* 338.08 [M+H]⁺, calculated MW 337.39; **¹H-NMR** (400 MHz, DMSO-*d*₆) d 8.53 (t, *J =* 5.8 Hz, 1 H), 7.86 (dd, *J =* 6.4, 2.0 Hz, 2 H), 7.65 ~ 7.61 (m, 3 H), 7.53 ~ 7.44 (m, 1 H), 7.33 (t, *J =* 9.6 Hz, 1 H), 7.25 (td, *J =* 7.6, 0.9 Hz, 1 H), 4.84 (s, 1 H), 3.24 (t, *J =* 6.0 Hz, 2 H), 1.97 ~ 1.92 (m, 3 H), 1.73 ~ 1.67 (m, 2 H), 1.16 (s, 3 H).

### Preparation Example 13: Synthesis of (R)-4-((2-fluorophenyl)ethynyl)-N-((tetrahydro-2H-pyran-3-yl)methyl)benzamide (Compound 13)

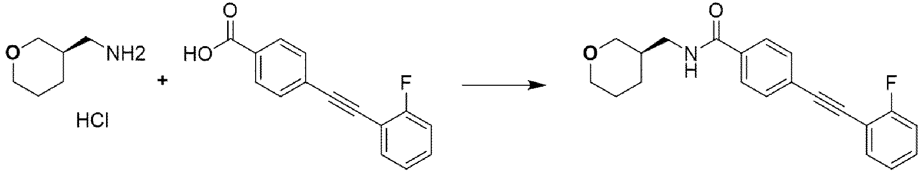

4-((2-Fluorophenyl)ethynyl)benzoic acid (0.050 g, 0.208 mmol) and (*R*)-(tetrahydro-2*H-*pyran-3-yl)methanamine hydrochloride (0.035 g, 0.229 mmol) as starting materials were used in a similar manner to Step 3 of Preparation Example 3 to obtain (*R*)-4-((2-fluorophenyl)ethynyl)-*N-*((tetrahydro-2*H*-pyran-3-yl)methyl)benzamide (0.055 g, 78.3%) as white solid: **LRMS** (ES) *m*/*z* 338.20 [M+H]⁺, calculated MW 337.39; **¹H-NMR** (400 MHz, DMSO-*d*₆) d 8.58 (t, *J =* 5.8 Hz, 1 H), 7.86 (dd, *J =* 6.6, 1.8 Hz, 2 H), 7.65 ~ 7.61 (m, 3 H), 7.44 ~ 7.35 (m, 1 H), 7.33 (td, *J =* 9.2, 0.8 Hz, 1 H), 7.25 (td, *J =* 7.6, 1.1 Hz, 1 H), 3.75 ~ 3.72 (m, 1 H), 3.69 (s, 1 H), 3.30 ~ 3.25 (m, 1 H), 3.15 ~ 3.05 (m, 3 H), 1.79 ~ 1.72 (m, 2 H), 1.57 ~ 1.52 (m, 1 H), 1.49 ~ 1.35 (m, 1 H), 1.26 ~ 1.21 (m, 1 H).

### Preparation Example 14: Synthesis of (S)-4-((2-fluorophenyl)ethynyl)-N-((tetrahydrofuran-3-yl)methyl)benzamide (Compound 14)

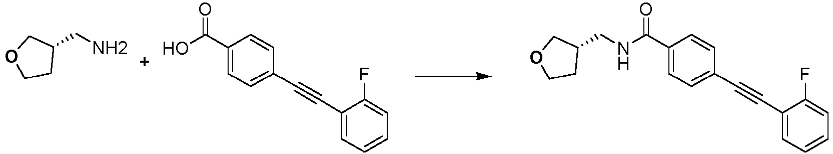

4-((2-Fluorophenyl)ethynyl)benzoic acid (0.070 g, 0.291 mmol) and (*S*)-(tetrahydrofuran-3-yl)methanamine (0.032 g, 0.321 mmol) as starting materials were used in a similar manner to Step 3 of Preparation Example 3 to obtain (S)-4-((2-fluorophenyl)ethynyl)-*N*-((tetrahydrofuran-3-yl)methyl)benzamide (0.089 g, 94.5%) as clear liquid: **LRMS** (ES) *m*/*z* 324.05 [M+H]⁺, calculated MW 323.37; **¹H-NMR** (400 MHz, DMSO-*d*₆) d 8.68 (*t, J =* 5.8 Hz, 1 H), 7.86 (dd, *J* = 6.8, 2.0 Hz, 2 H), 7.65 ~ 7.61 (m, 3 H), 7.50 ~ 7.43 (m, 1 H), 7.33 (td, *J=* 9.1, 1.1 Hz, 1 H), 7.25 (td, *J* = 7.7, 1.1 Hz, 1 H), 3.75 ~ 3.69 (m, 1 H), 3.68 ~ 3.62 (m, 1 H), 3.60 ~ 3.55 (m, 1 H), 3.45 ~ 3.40 (m, 1 H), 3.33 ~ 3.19 (m, 3 H), 1.95 ~ 1.87 (m, 1 H), 1.61 ~ 1.51 (m, 1 H).

### Preparation Example 15: Synthesis of 4-((4-fluorophenyl)ethynyl)-N-((tetrahydrofuran-3-yl)methyl)benzamide (Compound 15)

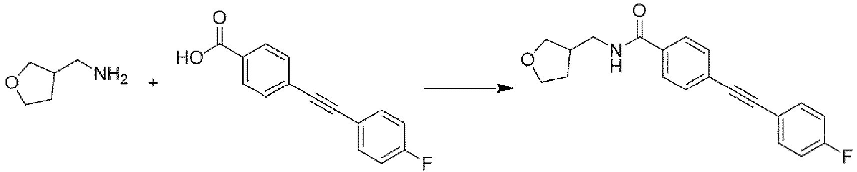

4-((4-Fluorophenyl)ethynyl)benzoic acid (0.070 g, 0.291 mmol) and (tetrahydrofuran-3-yl)methanamine (0.035 g, 0.350 mmol) as starting materials were used in a similar manner to Step 3 of Preparation Example 3 to obtain 4-((4-fluorophenyl)ethynyl)-N-((tetrahydrofuran-3-yl)methyl)benzamide (0.079 g, 83.8%) as ivory solid: **LRMS** (ES) *m*/*z* 324.14 [M+H]⁺, calculated MW 323.37; **¹H-NMR** (400 MHz, DMSO-*d*₆) d 8.64 (t, *J =* 5.8 Hz, 1 H), 7.85 (dd, *J* = 6.8, 2.0 Hz, 2 H), 7.64 ~ 7.58 (m, 4 H), 7.29 ~ 7.23 (m, 2 H), 3.73 ~ 3.68 (m, 1 H), 3.65 ~ 3.63 (m, 1 H), 3.61 ~ 3.55 (m, 1 H), 3.45 ~ 3.42 (m, 1 H), 3.22 ~ 3.15 (m, 3 H), 1.95 ~ 1.86 (m, 1 H), 1.60 ~ 1.52 (m, 1H).

### Preparation Example 16: Synthesis of 4-((2-fluorophenyl)ethynyl)-N-((3-methyloxetan-3-yl)methyl-d)benzamide (Compound 16)

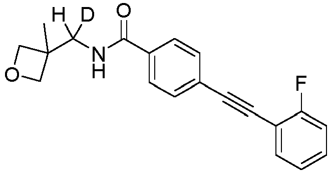

### Step 1: Synthesis of (3-methyloxetan-3-yl)methane-J-amine

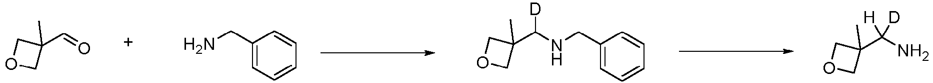

3-Methyloxetane-3-carbaldehyde (2.000 g, 19.976 mmol), phenylmethanamine (2.141 g, 19.976 mmol) and acetic acid (0.114 mL, 1.998 mmol) were dissolved in tetrahydrofuran (18 mL), and the resulting solution was stirred at room temperature for 1 hour. Sodium borodeuteride (1.254 g, 29.964 mmol) was added thereto and further stirred at the same temperature for 24 hours. A saturated aqueous sodium hydrogen carbonate solution was poured into the reaction mixture, followed by extraction with dichloromethane. The organic layer was washed with a saturated aqueous solution of ammonium chloride, dried over anhydrous magnesium sulfate, filtered and concentrated under reduced pressure to obtain *N-*benzyl-1-(3-methyloxetan-3-yl)methane-*d-*amine (3.800 g, 98.9%, light yellow oil): **LRMS** (ES) *m*/*z* 193.15 [M+H]⁺, calculated MW 192.28.

The obtained product, 10% Pd/C (600 mg) and ammonium formate (4.985 g, 79.051 mmol) were dissolved in methanol (20 mL) at room temperature. The resulting solution was stirred at 65°C for 14 hours, and then the temperature was lowered to room temperature to terminate the reaction. The reaction mixture was filtered through a Celite pad to remove the solid, and the solvent was removed from the filtrate under reduced pressure to obtain (3-methyloxetan-3-yl)methane-*d*-amine (1.280 g, 63.4%, yellow oil): **LRMS** (ES) *m*/*z* 102.97 [M+H]⁺, calculated MW 102.16.

### Step 2: Synthesis of 4-((2-fluorophenyl)ethynyl)-7V-((3-methyloxetan-3-yl)methyl-d)benzamide

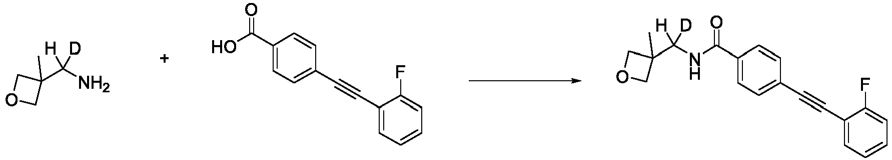

4-((2-Fluorophenyl)ethynyl)benzoic acid (3.010 g, 12.529 mmol) and (3-methyloxetan-3-yl)methane-d-amine (1.280 g, 12.529 mmol) as starting materials were used in a similar manner to Step 3 of Preparation Example 3 to obtain 4-((2-fluorophenyl)ethynyl)-*N*-((3-methyloxetan-3-yl)methyl-d)benzamide (1.750 g, 43.1%) as white solid: **LRMS** (ES) *m*/*z* 325.14 [M+H]⁺, calculated MW 324.37; **¹H-NMR** (400 MHz, DMSO-*d*₆) d 8.69 (d, *J =* 6.0 Hz, 1 H), 7.88 (dd, *J =* 7.0, 1.8 Hz, 2 H), 7.65 ~ 7.63 (m, 3 H), 7.51 ~ 7.45 (m, 1 H), 7.33 (m, 1 H), 7.25 (m, 1 H), 4.44 (d, *J =* 6.0 Hz, 2 H), 4.17 (d, *J =* 5.6 Hz, 2 H), 3.41 (d, *J =* 6.0 Hz, 1 H), 1.22 (s, 3 H).

### Preparation Example 17: Synthesis of (S)-4-((4-fluorophenyl)ethynyl)-N-((tetrahydrofuran-3-yl)methyl)benzamide (Compound 17)

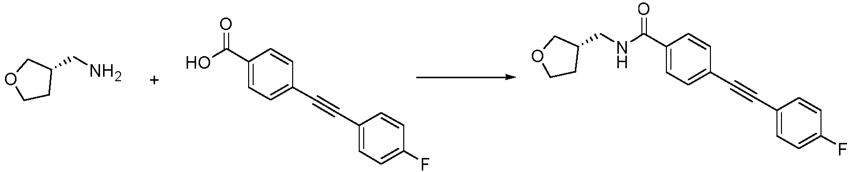

4-((4-Fluorophenyl)ethynyl)benzoic acid (0.150 g, 0.624 mmol) and (*S*)-(tetrahydrofuran-3-yl)methanamine (0.076 g, 0.749 mmol) as starting materials were used in a similar manner to Step 3 of Preparation Example 3 to obtain (*S*)-4-((4-fluorophenyl)ethynyl)-*N*-((tetrahydrofuran-3-yl)methyl)benzamide (0.152 g, 75.3% ) as yellow solid: **LRMS** (ES) *m*/*z* 324.08 [M+H]⁺, calculated MW 323.37; **¹H-NMR** (400 MHz, DMSO-*d*₆) d 8.66 (t, *J =* 5.6 Hz, 1 H), 7.85 (d, *J =* 8.4 Hz, 2 H), 7.63 ~ 7.60 (m, 4 H), 7.29 ~ 7.24 (m, 2 H), 3.73 ~ 3.68 (m, 1 H), 3.66 ~ 3.62 (m, 1 H), 3.60 ~ 3.55 (m, 1 H), 3.45 ~ 3.42 (m, 1 H), 3.29 ~ 3.12 (m, 3 H), 1.94 ~ 1.86 (m, 1 H), 1.60 ~ 1.52 (m, 1 H).

### Preparation Example 18: Synthesis of (R)-4-((2,4-difluorophenyl)ethynyl)-N-((tetrahydrofuran-3-yl)methyl)benzamide (Compound 18)

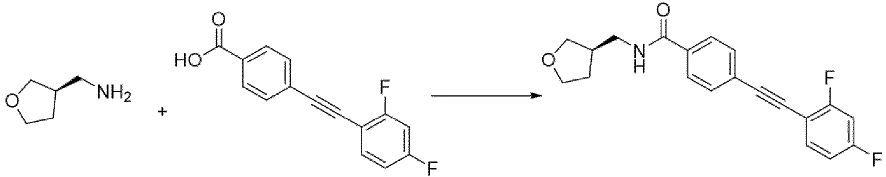

4-((2,4-Difluorophenyl)ethynyl)benzoic acid (0.200 g, 0.775 mmol) and (R)-(tetrahydrofuran-3-yl)methanamine (0.086 g, 0.852 mmol) as starting materials were used in a similar manner to Step 3 of Preparation Example 3 to obtain (*R*)-4-((2,4-difluorophenyl)ethynyl)-*N*-((tetrahydrofuran-3-yl)methyl)benzamide (0.230 g, 87.0%) as white solid: **LRMS** (ES) *m*/*z* 342.18 [M+H]⁺, calculated MW 341.36; **¹H-NMR** (400 MHz, DMSO-*d*₆) d 8.66 (t, *J =* 5.8 Hz, 1 H), 7.86 (d, *J =* 8.8 Hz, 2 H), 7.74 ~ 7.68 (m, 1 H), 7.63 (d, *J =* 14.8 Hz, 2 H), 7.47 ~ 7.38 (m, 1 H), 7.19 ~ 7.15 (m, 1 H), 3.73 ~ 3.69 (m, 1 H), 3.67 ~ 3.63 (m, 1 H), 3.61 ~ 3.55 (m, 1 H), 3.45 ~ 3.42 (m, 1 H), 3.26 ~ 3.17 (m, 2 H), 3.13 (d, *J =* 5.6 Hz, 1 H), 1.96 ~ 1.84 (m, 1 H), 1.60 ~ 1.52 (m, 1 H).

### Preparation Example 19: Synthesis of (S)-4-((2,4-difluorophenyl)ethynyl)-N-((tetrahydrofuran-3-yl)methyl)benzamide (Compound 19)

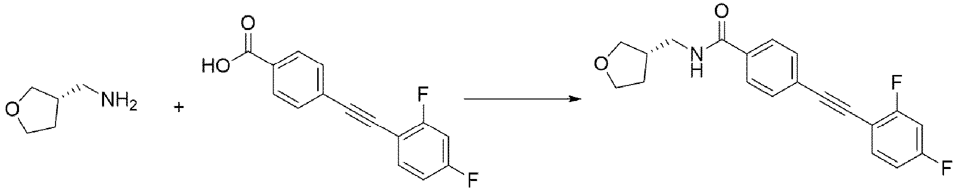

4-((2,4-Difluorophenyl)ethynyl)benzoic acid (0.060 g, 0.232 mmol) and (*S*)-(tetrahydrofuran-3-yl)methanamine (0.026 g, 0.256 mmol) as starting materials were used in a similar manner to Step 3 of Preparation Example 3 to obtain (S)-4-((2,4-difluorophenyl)ethynyl)-N ((tetrahydrofuran-3-yl)methyl)benzamide (0.073 g, 92.0%) as white solid: **LRMS** (ES) *m*/*z* 342.18 [M+H]⁺, calculated MW 341.36; **¹H-NMR** (400 MHz, DMSO-*d*₆) d 8.66 (t, *J =* 5.6 Hz, 1 H), 7.86 (dd, *J =* 6.6, 2.2 Hz, 2 H), 7.71 (q, *J =* 7.9 Hz, 1 H), 7.62 (d, *J =* 8.8 Hz, 2 H), 7.43 (dt, *J* = 16.4, 4.8 Hz, 1 H), 7.18 (d, *J =* 26.0 Hz, 1 H), 3.73 ~ 3.63 (m, 2 H), 3.61 ~ 3.42 (m, 2 H), 3.26 - 3.16 (m, 3 H), 1.95 ~ 1.86 (m, 1 H), 1.61 ~ 1.52 (m, 1 H).

### Preparation Example 20: Synthesis of (S)-4-((2,4-dffluorophenyl)ethynyl)-N-((tetrahydro-2H-pyran-3-yl)methyl)benzamide (Compound 20)

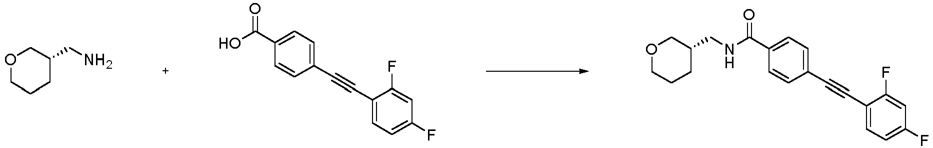

(*S*)-(tetrahydro-2*H*-pyran-3-yl)methanamine hydrochloride (0.050 g, 0.330 mmol) and 4-((2,4-difluorophenyl)ethynyl)benzoic acid (0.102 g, 0.396 mmol) as starting materials were used in a similar manner to Step 3 of Preparation Example 3 to obtain (*S*)-4-((2,4-difluorophenyl)ethynyl)-*N*-((tetrahydro-2*H*-pyran-3-yl)methyl)benzamide (0.050 g, 42.7%) as white solid: **LRMS** (ES) *m*/*z* 356.18 [M+H]⁺, calculated MW 355.38; **¹H-NMR** (400 MHz, CD₃OD) d 8.54 (bs, 1 H), 7.81 (d, *J =* 6.4 Hz, 2 H), 7.58 (d, *J =* 7.2 Hz, 3 H), 6.98 ~ 7.05 (m, 2 H), 3.79 ~ 3.88 (m, 2 H), 3.42 (m, 1 H), 3.25 (m, 2 H), 1.89 (m, 2 H), 1.56 ~ 1.64 (m, 2 H), 1.30 ~ 1.32 (m, 2 H).

### Preparation Example 21: Synthesis of N-((3,3-difluorocyclobutyl)methyl)-4-((2,4-difluorophenyl)ethynyl)benzamide (Compound 21)

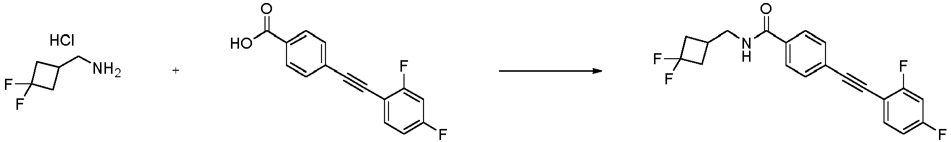

(3,3-Difluorocyclobutyl)methanamine hydrochloride (0.050 g, 0.317 mmol) and 4-((2,4-difluorophenyl)ethynyl)benzoic acid (0.098 g, 0.381 mmol) as starting materials were used in a similar manner to Step 3 of Preparation Example 3 to obtain *N*-((3,3-difluorocyclobutyl)methyl)-4-((2,4-difluorophenyl)ethynyl)benzamide (0.049 g, 42.7%) as white solid: **LRMS** (ES) *m*/*z* 362.19 [M+H]⁺, calculated MW 361.34; **¹H-NMR** (400 MHz, CD₃OD) d 7.75 ~ 7.81 (m, 2 H), 7.54 ~ 7.59 (m, 3 H), 7.02 (m, 2 H), 3.48 (d, *J =* 6.0 Hz, 2 H), 3.28 ~ 3.29 (m, 1 H), 2.62 (m, 2 H), 2.32 (m, 2 H).

### Preparation Example 22: Synthesis of 4-((2,4-difluorophenyl)ethynyl)-N-((3-ethyloxetan-3-yl)methyl)benzamide (Compound 22)

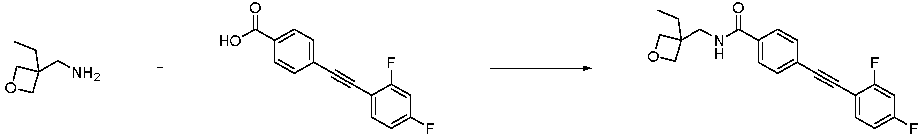

(3-Ethyloxetan-3-yl)methanamine (0.050 g, 0.434 mmol) and 4-((2,4-difluorophenyl)ethynyl)benzoic acid (0.135 g, 0.521 mmol) as starting materials were used in a similar manner to Step 3 of Preparation Example 3 to obtain 4-((2,4-difluorophenyl)ethynyl)-*N-*((3-ethyloxetan-3-yl)methyl)benzamide (0.070 g, 45.4%) as white solid: **LRMS** (ES) *m*/*z* 356.18 [M+H]⁺, calculated MW 355.38; **¹H-NMR** (400 MHz, CD₃OD) d 7.82 (d, *J =* 8.4 Hz, 2 H), 7.58 (d, *J =* 8.4 Hz, 2 H), 7.54 ~ 7.60 (m, 1 H), 6.96 ~ 7.07 (m, 2 H), 4.56 (d, *J =* 6.4 Hz, 2 H), 4.39 (d, *J =* 6.0 Hz, 2 H), 3.59 (s, 2 H), 1.74 (q, *J =* 7.5 Hz, 2 H), 0.97 (t, *J =* 7.4 Hz, 3 H).

### Preparation Example 23: Synthesis of 4-((2,4-difluorophenyl)ethynyl)-N-((3-fluorooxetan-3-yl)methyl)benzamide (Compound 23)

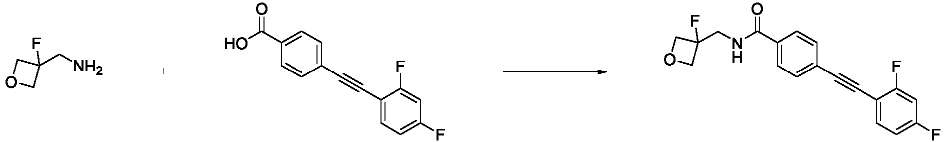

(3-Fluorooxetan-3-yl)methanamine (0.050 g, 0.476 mmol) and 4-((2,4-difluorophenyl)ethanyl)benzoic acid (0.147 g, 0.571 mmol) as starting materials were used in a similar manner to Step 3 of Preparation Example 3 to obtain 4-((2,4-difluorophenyl)ethynyl)-*N-*((3-fluorooxetan-3-yl)methyl)benzamide (0.090 g, 54.8%) as white solid: **LRMS** (ES) *m*/*z* 346.21 [M+H]⁺, calculated MW 345.32; **¹H-NMR** (400 MHz, CD₃OD) d 7.83 (d, *J =* 8.0 Hz, 2 H), 7.58 (d, *J =* 8.4 Hz, 2 H), 7.54 ~ 7.60 (m, 1 H), 7.04 (td, *J =* 9.4, 2.4 Hz, 1 H), 6.99 (td, *J =* 8.6, 2.0 Hz, 1 H), 4.74 (s, 2 H), 4.69 (s, 2 H), 3.90 (d, *J =* 20.0 Hz, 2 H).

### Preparation Example 24: Synthesis of 4-((2,4-difluorophenyl)ethynyl)-N-((4-methyltetrahydro-2H-pyran-4-yl)methyl)benzamide (Compound 24)

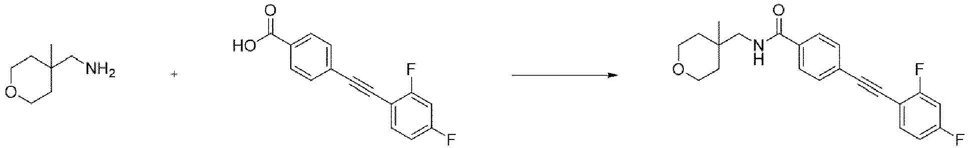

(4-Methyltetrahydro-2*H*-pyran-4-yl)methanamine (0.050 g, 0.387 mmol) and 4-((2,4-difluorophenyl)ethynyl)benzoic acid (0.120 g, 0.464 mmol) as starting materials were used in a similar manner to Step 3 of Preparation Example 3 to obtain 4-((2,4-difluorophenyl)ethynyl)-*N-*((4-methyltetrahydro-2*H*-pyran-4-yl)methyl)benzamide (0.042 g, 29.4%) as white solid: **LRMS** (ES) *m*/*z* 370.25 [M+H]⁺, calculated MW 369.41; **¹H-NMR** (400 MHz, CD₃OD) d 7.82 (d, *J =* 8.0 Hz, 2 H), 7.59 (d, *J =* 8.8 Hz, 2 H), 7.55 ~ 7.61 (m, 1 H), 6.97 ~ 7.09 (m, 2 H), 3.58 ~ 3.76 (m, 4 H), 3.26 ~ 3.35 (m, 2 H), 1.52 ~ 1.57 (m, 2 H), 1.34 (m, 2 H), 1.02 (s, 3 H).

### Preparation Example 25: Synthesis of 4-((2,4-difluorophenyl)ethynyl)-N-(2-(tetrahydro-2H-pyran-4-yl)ethyl)benzamide (Compound 25)

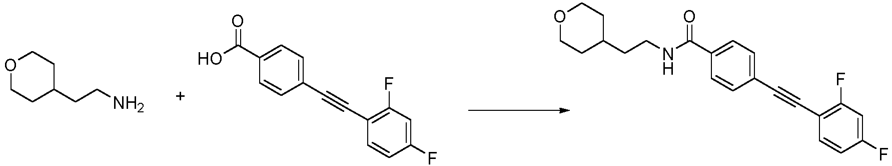

2-(Tetrahydro-2*H*-pyran-4-yl)ethan-1-amine (0.042 g, 0.325 mmol) and 4-((2,4-difluorophenyl)ethynyl)benzoic acid (0.070 g, 0.271 mmol) mmol) as starting materials were used in a similar manner to Step 3 of Preparation Example 3 to obtain 4-((2,4-difluorophenyl)ethynyl)-*N*-(2-(tetrahydro-2*H*-pyran-4-yl)ethyl)benzamide (0.050 g, 49.9%) as white solid: **LRMS** (ES) *m*/*z* 370.25 [M+H]⁺, calculated MW 369.41; **¹H-NMR** (400 MHz, DMSO-*d*₆) d 8.53 (t, *J =* 5.6 Hz, 1 H), 7.85 (d, *J =* 8.8 Hz, 2 H), 7.68 ~ 7.73 (m, 1 H), 7.62 (d, *J =* 8.8 Hz, 2 H), 7.43 (td, *J =* 9.7, 2.5 Hz, 1 H), 7.15 ~ 7.19 (m, 1 H), 3.78 (dd, *J =* 10.8, 3.2 Hz, 2 H), 3.19 ~ 3.29 (m, 4 H), 1.58 (d, *J =* 16.0 Hz, 2 H), 1.47 ~ 1.54 (m, 1 H), 1.43 (q, *J =* 7.1 Hz, 2 H), 1.12 (ddd, *J =* 23.5, 12.1, 4.1 Hz, 2 H).

### Preparation Example 26: Synthesis of 4-((4-fluorophenyl)ethynyl)-N-((1-hydroxycyclohexyl)methyl)benzamide (Compound 26)

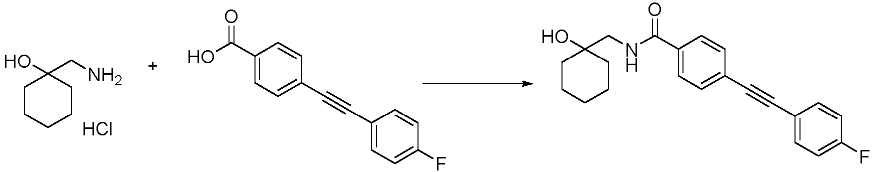

1-(Aminomethyl)cyclohexan-1-ol hydrochloride (0.050 g, 0.300 mmol) and 4-((4-fluorophenyl)ethynyl)benzoic acid (0.060 g, 0.250 mmol) as starting materials were used in a similar manner to Step 3 of Preparation Example 3 to obtain 4-((4-fluorophenyl)ethynyl)-N-((1-hydroxycyclohexyl)methyl)benzamide (0.054 g, 61.5%) as yellow solid: **LRMS** (ES) *m*/*z* 352.28 [M+H]⁺, calculated MW 351.42; **¹H-NMR** (400 MHz, DMSO-*d*₆) d 8.25 (t, *J =* 6.0 Hz, 1 H), 7.87 (d, *J =* 8.8 Hz, 2 H), 7.59 ~ 7.64 (m, 4 H), 7.26 (t, *J =* 9.0 Hz, 2 H), 4.32 (s, 1 H), 3.23 (d, *J =* 5.6 Hz, 2 H), 1.47 ~ 1.55 (m, 2 H), 1.36 ~ 1.42 (m, 4 H), 1.12 ~ 1.36 (m, 4 H).

### Preparation Example 27: Synthesis of 4-((4-fluorophenyl)ethynyl)-N-((1-hydroxycyclobutyl)methyl)benzamide (Compound 27)

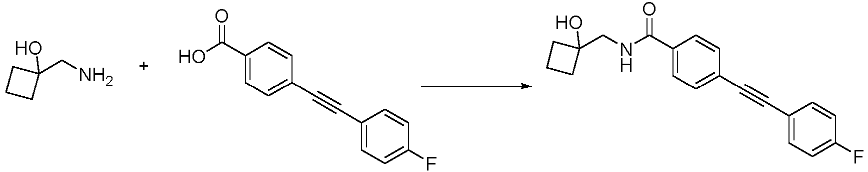

1-(Aminomethyl)cyclobutan-1-ol (0.030 g, 0.300 mmol) and 4-((4-fluorophenyl)ethynyl)benzoic acid (0.060 g, 0.250 mmol) as starting materials were used in a similar manner to Step 3 of Preparation Example 3 to obtain 4-((4-fluorophenyl)ethynyl)-N-((1-hydroxycyclobutyl)methyl)benzamide (0.049 g, 60.7%) as ivory solid: **LRMS** (ES) m/z 324.28 [M+H]⁺, calculated MW 323.37; **¹H-NMR** (400 MHz, DMSO-*d*₆) d 8.39 (t, *J =* 5.8 Hz, 1 H), 7.88 (dd, *J =* 6.4, 1.6 Hz, 2 H), 7.59 ~ 7.64 (m, 4 H), 7.23 ~ 7.29 (m, 2 H), 5.15 (s, 1 H), 3.39 (d, *J =* 6.0 Hz, 2 H), 1.98 ~ 2.04 (m, 2 H), 1.83 ~ 1.91 (m, 2 H), 1.55 ~ 1.64 (m, 1 H), 1.38 ~ 1.49 (m, 1 H).

### Preparation Example 28: Synthesis of 4-((2,4-difluorophenyl)ethynyl)-N-((1-hydroxycyclohexyl)methyl)benzamide (Compound 28)

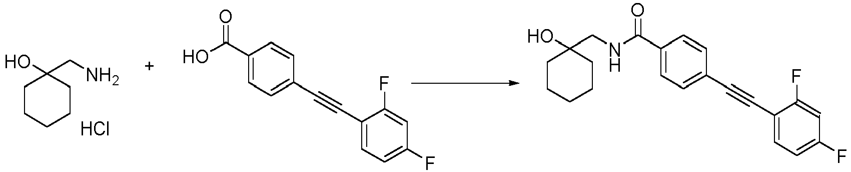

1-(Aminomethyl)cyclohexan-1-ol hydrochloride (0.046 g, 0.279 mmol) and 4-((2,4-difluorophenyl)ethynyl)benzoic acid (0.060 g, 0.232 mmol) as starting materials were used in a similar manner to Step 3 of Preparation Example 3 to obtain 4-((2,4-difluorophenyl)ethynyl)-N-((1-hydroxycyclohexyl)methyl)benzamide (0.057 g , 66.4%) as ivory solid: **LRMS** (ES) *m*/*z* 370.23 [M+H]⁺, calculated MW 369.41; **¹H-NMR** (400 MHz, DMSO-*d*₆) d 8.25 (t, *J=* 6.0 Hz, 1 H), 7.89 (dd, *J =* 6.4, 2.0 Hz, 2 H), 7.68 ~ 7.74 (m, 1 H), 7.62 (dd, *J =* 6.4, 2.0 Hz, 2 H), 7.42 (td, *J =* 9.6, 2.0 Hz, 1 H), 7.15 ~ 7.19 (m, 1 H), 4.31 (s, 1 H), 3.24 (d, *J =* 6.0 Hz, 2 H), 1.46 ~ 1.55 (m, 2 H), 1.39 ~ 1.43 (m, 4 H), 1.13 ~ 1.36 (m, 4 H).

### Preparation Example 29: Synthesis of 4-((2,4-difluorophenyl)ethynyl)-N-((1-hydroxycyclobutyl)methyl)benzamide (Compound 29)

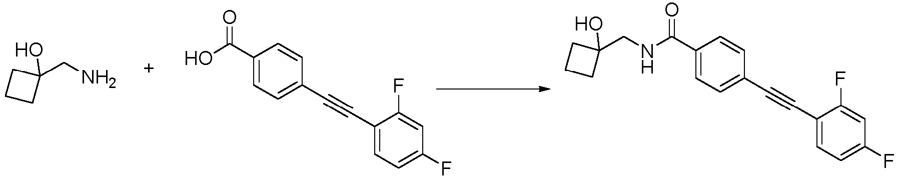

1-(Aminomethyl)cyclobutan-1-ol (0.028 g, 0.279 mmol) and 4-((2,4-difluorophenyl)ethynyl)benzoic acid (0.060 g, 0.232 mmol) as starting materials were used in a similar manner to Step 3 of Preparation Example 3 to obtain 4-((2,4-difluorophenyl)ethynyl)-N-((1-hydroxycyclobutyl)methyl)benzamide (0.045 g, 56.7%) as ivory solid: **LRMS** (ES) *m*/*z* 342.24 [M+H]⁺, calculated MW 341.36; **¹H-NMR** (400 MHz, DMSO-*d*₆) d 8.40 (t, *J =* 5.8 Hz, 1 H), 7.89 (dd, *J =* 6.8, 2.0 Hz, 2 H), 7.71 (q, *J =* 8.0 Hz, 1 H), 7.62 (dd, *J =* 6.8, 1.6 Hz, 2 H), 7.42 (td, *J =* 9.0, 3.6 Hz, 1 H), 7.15 ~ 7.19 (m, 1 H), 5.10 (s, 1 H), 3.39 (d, *J =* 6.0 Hz, 2 H), 1.98 ~ 2.04 (m, 2 H), 1.83 ~ 1.91 (m, 2 H), 1.55 ~ 1.64 (m, 1 H), 1.38 ~ 1.50 (m, 1 H).

### Preparation Example 30: Synthesis of N-(cyclobutylmethyl)-4-((2,4-difluorophenyl)ethynyl)benzamide (Compound 30)

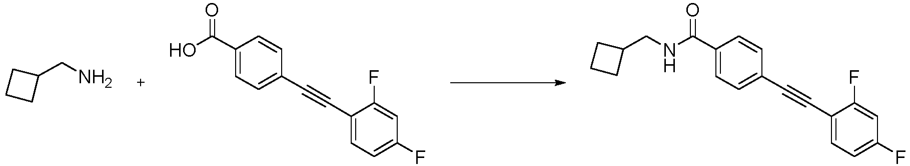

Cyclobutylmethanamine (0.030 g, 0.352 mmol) and 4-((2,4-difluorophenyl)ethynyl)benzoic acid (0.109 g, 0.423 mmol) as starting materials were used in a similar manner to Step 3 of Preparation Example 3 to obtain *N*-(cyclobutylmethyl)-4-((2,4-difluorophenyl)ethynyl)benzamide (0.075 g, 65.4%) as white solid: **LRMS** (ES) *m*/*z* 326.26 [M+H]⁺, calculated MW 325.36; **¹H-NMR** (400 MHz, DMSO-*d*₆) d 8.52 (t, *J =* 5.8 Hz, 1 H), 7.85 (d, *J =* 8.4 Hz, 2 H), 7.70 (q, *J =* 7.7 Hz, 1 H), 7.61 (d, *J =* 8.8 Hz, 2 H), 7.42 (td, *J =* 9.7, 2.7 Hz, 1 H), 7.19 ~ 7.14 (m, 1 H), 3.25 (d, *J =* 6.0 Hz, 2 H), 2.53 ~ 2.49 (m, 1 H), 1.98 ~ 1.92 (m, 2 H), 1.83 ~ 1.75 (m, 2 H), 1.72 ~ 1.65 (m, 2 H).

### Experimental Example 1: Fluorescence-based Ca²⁺ mobilization assay

In order to measure the activity on the mGluR5, an experiment was carried out to check the change in the intracellular Ca²⁺ level using the HEK293 cell line permenantly overexpressing the mGluR5. The cells in the cell culture medium were aliquoted into a 384-well plate coated with poly-D-lysine and cultured in a 37°C incubator supplied with 5% CO₂. The next day, the medium was removed and a dye loading buffer-in which a reagent capable of measuring Ca²⁺ was dissolved-was added, and then incubated at 37°C for 60 minutes. The test compounds were dissolved in 100% DMSO at a final concentration of 10 mM to make a stock solution. The starting concentration of 10 µM was prepared from the stock solution, followed by serial dilutions to prepare working solutions (test compound working solutions). The test compound working solutions were added to the cells that were incubated in the dye loading buffer, which were maintained for 30 minutes at room temperature in a light-shielding state before measurement. The Ca²⁺ level change induced by L-glutamate at EC₈₀ concentration was measured for 2 minutes using FLIPR Tetra (MDS Analytical Technologies). The fluorescent Ca²⁺ signal values in the presence of the test compounds were normalized to the fluorescent singal elicited by EC₈₀ concentration of L-glutamate as 100% and that elicited by vehicle as 0% to estimate % inhibition of the test compounds. The efficacy of the test compounds was calculated as IC₅₀ values and represented in Table 2. (+: 500-1,000 nM, ++: 100-500 nM, +++: less than 100 nM).

### Experimental Example 2: HTRF-based IP1 accumulation assay

The 5-HT_{2A} receptor antagonistic effect of the test compounds was confirmed by measuring the production amount of IP1 using the HEK293 cell line permanently overexpressing the human 5-HT_{2A} receptor. The test compounds were dissolved in 100% DMSO to a final concentration of 10 mM. The starting concentration of 10 µM was prepared from the stock solution, followed by serial dilutions to prepare working solutions in a buffer containing lithium chloride (LiCl). The cells and compounds prepared in the test buffer were placed in a 96-well plate and incubated at room temperature for 10 minutes. Serotonin of EC₈₀ concentration was added, followed by incubation at 37°C for 30 minutes in a light-shielding state. To measure IP1 accumulation in cells, IP1-d2 acceptor and IP1-Cryptate donor-which are HTRF-based substances for fluorescence signals-were added and incubated for 1 hour at room temperature in a light-shielded state. The fluorescent emission signals of acceptor and donor were detected, and HTRF ratio values were substracted by mean value of serotonin control. The percentage of activation was calculated by normalization of the serotonin control (0%) and IP1 control (100%). The IC₅₀ values of the test compounds are represented in Table 2. (+: 500-1,000 nM, ++: 100-500 nM, +++: less than 100 nM).

**[Table 2]**

| **Compound No.** | **Human 5-HT_{2A}R (IC₅₀, nM)** | **Human mGluR5 (IC₅₀, nM)** | **Compound No.** | **Human 5-HT_{2A}R (IC₅₀, nM)** | **Human mGluR5 (IC₅₀, nM)** |
|---|---|---|---|---|---|
| 1 | ++ | +++ | 16 | ++ | +++ |
| 2 | +++ | +++ | 17 | +++ | ++ |
| 3 | ++ | +++ | 18 | ++ | ++ |
| 4 | ++ | +++ | 19 | +++ | ++ |
| 5 | +++ | + | 20 | ++ | ++ |
| 6 | ++ | +++ | 21 | +++ | ++ |
| 7 | ++ | +++ | 22 | +++ | ++ |
| 8 | + | +++ | 23 | +++ | + |
| 9 | + | ++ | 24 | ++ | +++ |
| 10 | + | + | 25 | +++ | ++ |
| 11 | ++ | +++ | 26 | ++ | +++ |
| 12 | ++ | +++ | 27 | +++ | +++ |
| 13 | ++ | +++ | 28 | ++ | + |
| 14 | ++ | ++ | 29 | +++ | ++ |
| 15 | ++ | ++ | 30 | ++ | ++ |

### Example 1: Efficacy study for the treatment of drug addiction using animal model

Morphine is a narcotic opioid with powerful analgesic effects. Morphine is prescribed and used to relieve severe pain, but its use is limited due to various side effects (Dowell et al., 2016). Specifically, the addictive potential of morphine, which are serious side effects that cause drug dependence and withdrawal symptoms, makes its clinical use difficult (Kim et al., 2016).

Drug self-administration is a valid preclinical research method that can confirm the addictive potential of a drug (Panlilio & Goldberg, 2007), and many previous studies have proven that morphine is an addictive substance that induces self-administration (Ma et al., 2018; Ucha et al., 2019).

As experimental animals, male Sprague-Dawley rats (230-250 g) were purchased from OrientBio (Seongnam-si, Korea) and provided by the Laboratory Animal Research Center of Sungkyunkwan University. Rats were housed in a breeding room maintained at 22 ± 2°C under a 12-hour light/dark cycle. Each rat was housed alone in a separate cage from acquisition to the end of the experiment and had *ad libitum* access to food and water during the experiment except for the food training period. All experiments were performed during the light cycle between 10:00 and 18:00.

Intravenous self-administration (IVSA) experiments were performed according to previously reported experimental methods (Ma et al., 2017; Ucha et al., 2019) with minor modifications.

During the experimental session, each rat was placed in an operant test chamber (Med Associates Inc., St Albans, VT) equipped with two response levers, and a fixed ratio of 1 (FR1), which required one press of the active lever to obtain one injection of morphine, was used. To facilitate operant responding on the lever, rats were trained to obtain a 45 mg food pellet by pressing the active lever. Food was withheld for 12 hours before training began, and training was conducted in daily 1-hour sessions until the acquisition criterion (80 food pellets for 3 consecutive days) was met. Rats that met the criteria through training underwent jugular vein catheter insertion, and after a 7-day recovery period, drug self-administration experiments began.

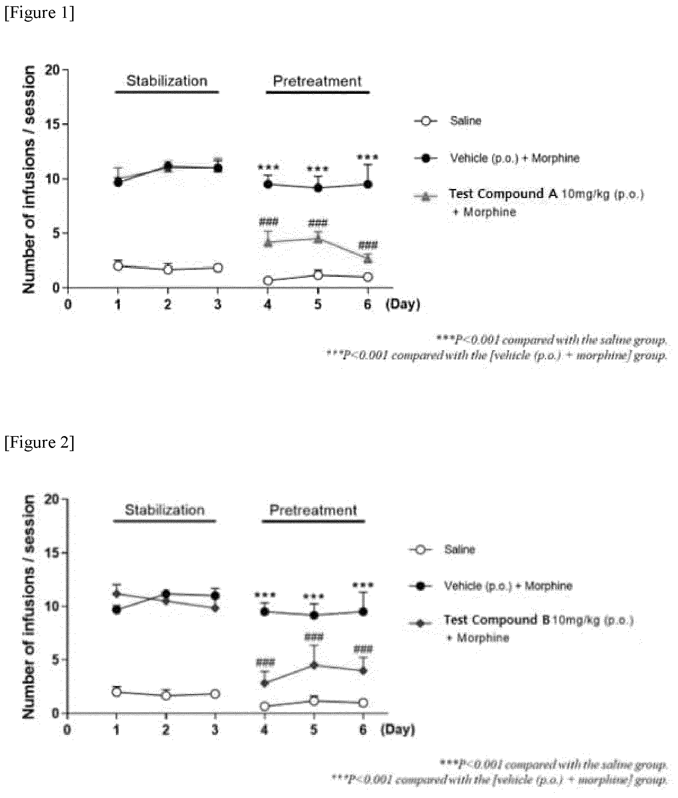

To investigate the therapeutic effect of the test compounds on morphine addiction, rats recovering from surgery were trained to intravenous self-administration (IVSA) of morphine for seven (7) consecutive days, 2 hours per session, using a fixed ratio of 1 (FR1) (0.1 mg/kg/infusion, 0.1 mL delivered within 4 seconds). After the rats showed a stable response (less than 20% variation in daily drug infusion over three (3) consecutive sessions), the effects of the test compounds on the maintenance of morphine IVSA were tested for three (3) consecutive days. As the test compounds, Compound 6 (4-((2-fluorophenyl)ethynyl)-N-((3-methyloxetan-3-yl)methyl)benzamide)(hereinafter referred to as "Test Compound A"), Compound 9 ((R)-4-((2-fluorophenyl)ethynyl)-N-((tetrahydrofuran-3-yl)methyl)benzamide)(hereinafter referred to as "Test Compound B") and Compound 1 (4-((2-fluorophenyl)ethynyl)-N-((tetrahydro-2H-pyran-4-yl)methyl)benzamide)(hereinafter referred to as "Test Compound C") were used. Test Compounds A, B, and C were dissolved in water containing 0.5% HPMC (hydroxypropyl methylcellulose) and 1% Tween 80, and were administered orally at doses of 10, 10, and 100 mg/kg, respectively, 2 hours before morphine IVSA. The treatment effects on the maintenance of morphine IVSA were measured, and the results are represented in Figures 1 to 3, respectively.

As shown in Figures 1 to 3, the control group pretreated with the solvent alone continued to maintain morphine self-administration, whereas the groups pretreated with test compounds significantly reduced the number of injections and active lever pressings, thereby suppressing the maintenance of morphine self-administration. These results confirm the effectiveness of the test compounds in treating morphine addiction.

### Example 2: Efficacy study for the prevention of drug addiction using animal model

The experimental animals and the experimental methods for intravenous self-administration were performed in a similar manner to Example 1, and Test Compounds A and C were used as the test compounds.

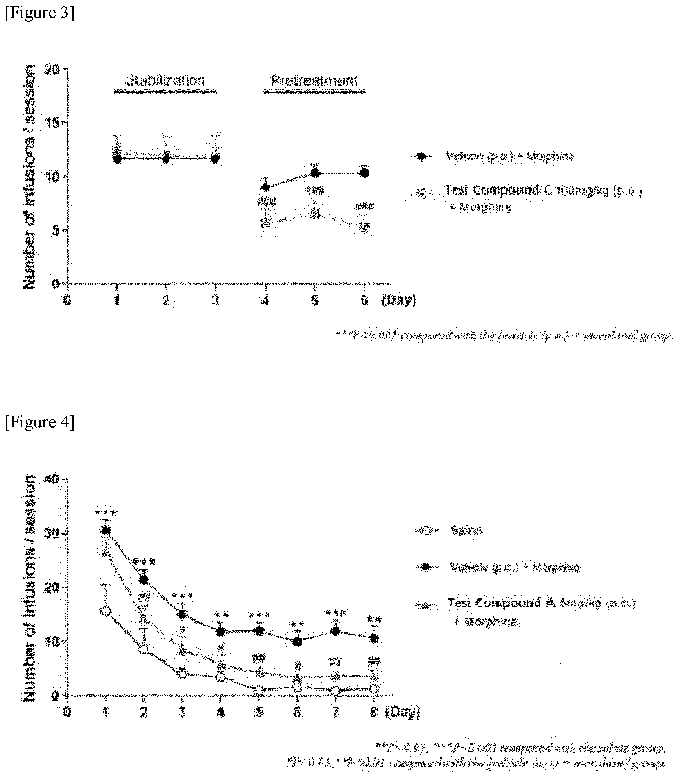

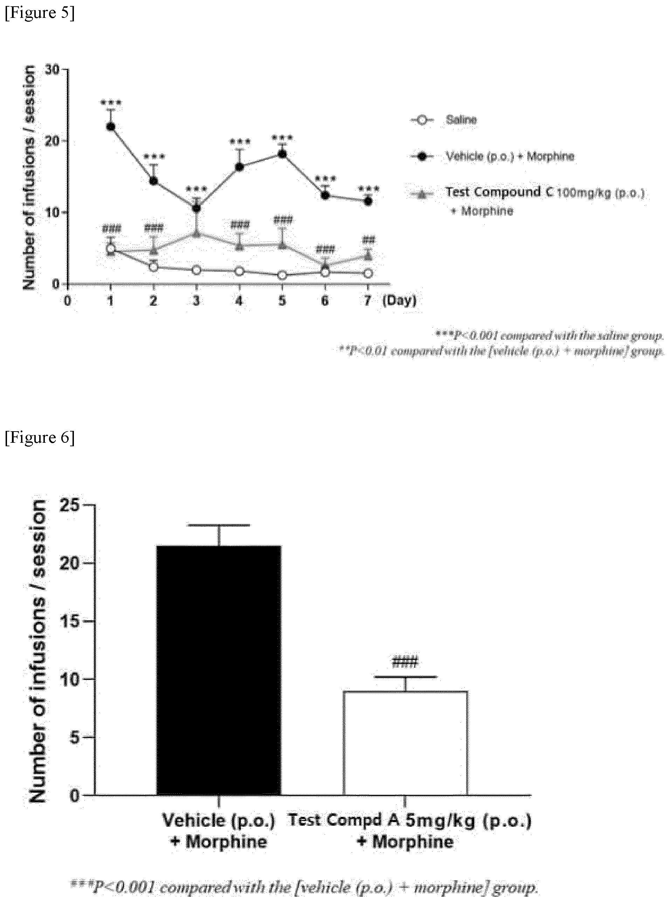

To investigate the preventive effect of the test compounds on morphine addiction, rats recovering from surgery were subjected to morphine intravenous self-administration (IVSA) for eight (8) consecutive days, with 2-hours sessions each day, using a fixed ratio of 1 (FR1) (0.1 mg/kg/infusion, 0.1 mL delivered within 4 seconds). Test compounds A and C were dissolved in water containing 0.5% HPMC (hydroxypropyl methylcellulose) and 1% Tween 80, and administered orally at doses of 5 mg/kg and 100 mg/kg, respectively, 2 hours before morphine IVSA. The preventive effects on acquisition of morphine IVSA were measured, and the results are represented in Figures 4 and 5.

As shown in Figures 4 and 5, the control group pretreated with solvent only maintained a high number of injections indicating morphine self-administration was acquired. However, when pretreated with Test Compounds A and C, the number of injections was significantly lower, and the acquisition of morphine self-administration was suppressed. These results confirm that the use of Test Compounds A and C alongside morphine can prevent morphine addiction when morphine is used as a pain suppressant.

### Example 3: Efficacy study on the prevention of relapse in drug addiction using animal model

### 3-1: Efficacy study for the prevention of relapse in morphine addiction model

Relapse of drug addiction is one of the major problems that appears during the withdrawal process after long-term exposure to drugs and is characterized by strong drug-seeking behavior (Dowell, Haegerich & Chou, 2016). Therefore, the prevention of relapse is necessary to treat drug abuse. In human addicts and animal models of recovery, re-exposure to the drug or cues related to the drug-taking environment can trigger drug craving and relapse (Lee et al., 2012; Ma et al., 2017).

The experimental animals and the experimental methods for intravenous self-administration were performed in a similar manner to Example 1, and the following test was conducted to investigate the inhibitory effect of the test compound on morphine reinstatement induced by a priming injection of morphine after a withdrawal period in morphine-addicted animals. Rats recovering from surgery were subjected to morphine intravenous self-administration (IVSA) training for at least eight (8) consecutive days, with 2-hour sessions each day, using a fixed ratio of 1 (FR1) (1 mg/kg/infusion, 0.1 mL delivered within 4 seconds). After the rats showed a stable response (less than 20% variation in daily drug infusion over three (3) consecutive sessions), a withdrawal period was given without any intervention for one (1) week (Lee et al., 2012). To test reinstatement by morphine priming, rats were injected subcutaneously with morphine (1.0 mg/kg, s.c.) 10 minutes before the recovery session. The dose and treatment method of morphine priming showed stable reinstatement, which is consistent with a previously published study (Swain, et. al., 2020). During the reinstatement test, rats were given saline instead of morphine when they pressed the active lever. Test Compound A, used as the test compound, was dissolved in water containing 0.5% HPMC (hydroxypropyl methylcellulose) and 1% Tween 80, and administered orally at a dose of 5 mg/kg, 2 hours before the reinstatement session. The effects on preventing the recurrence of morphine addiction were measured, and the results are represented in Figure 6.

As shown in Figure 6, when pretreated with the test compound, the number of drug injections by pressing the active lever was significantly reduced, indicating the inhibitory effect against morphine reinstatement.

### 3-2: Efficacy study for the prevention of relapse in cocaine addiction model

The experimental animals and the experimental methods for intravenous self-administration were performed in a similar manner to Example 1, and the following test was conducted to investigate the inhibitory effect of the test compounds on cocaine reinstatement induced by a priming injection of cocaine after a withdrawal period in cocaine-addicted animals.

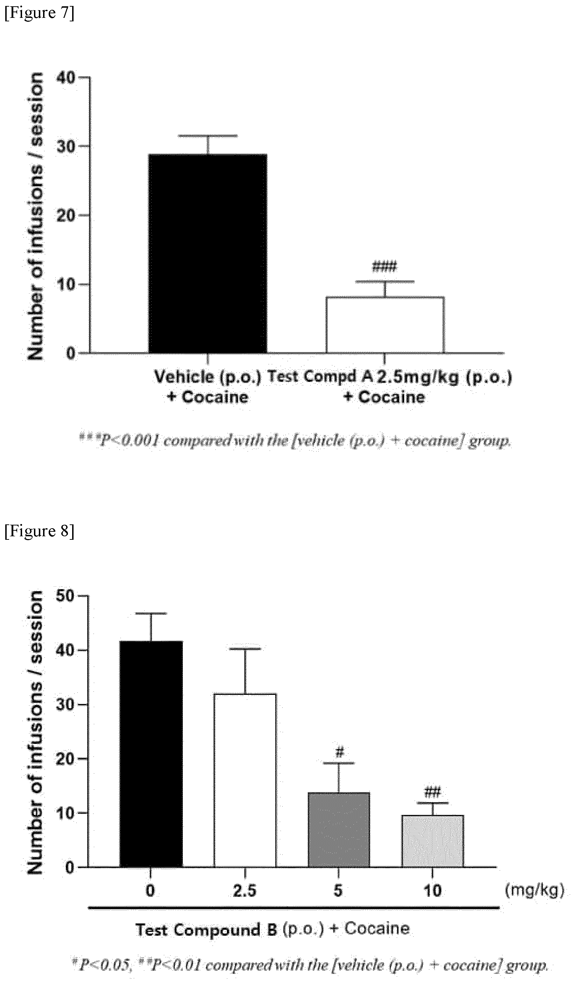

In the case of a test about the inhibitory effect of Test Compound A on cocaine reinstatement, rats recovering from surgery were subjected to cocaine intravenous self-administration (IVSA) training for ten (10) consecutive days, with 2-hour sessions each day, using a fixed ratio of 1 (FR1) (0.5 mg/kg/infusion, 0.1 mL delivered within 4 seconds). In the case of a test about the inhibitory effect of Test Compound B on cocaine reinstatement, rats recovering from surgery were subjected to cocaine intravenous self-administration (IVSA) training for five (5) consecutive days, with 2-hour sessions each day, using a fixed ratio of 1 (FR1), followed by the same amount of cocaine intravenous self-administration (IVSA) training for seven (7) consecutive days, with 2-hour sessions each day, using a fixed ratio of 2 (FR2). Once the cocaine self-administration response was stably established, cocaine administration was stopped the next day, and a withdrawal period was given without any intervention for one (1) week. When the cocaine self-administration response was stably extinguished, cocaine was administered intraperitoneally (10 mg/kg, i.p.) to test reinstatement by cocaine priming. As the test compounds, Test Compounds A and B were dissolved in water containing 0.5% HPMC (hydroxypropyl methylcellulose) and 1% Tween 80, and administered orally at a dose of 2.5 mg/kg, and at doses of 2.5, 5 and 10 mg/kg, respectively, 1 hour before the reinstatement session. The effects on preventing recurrence of cocaine addiction were measured, and the results are represented in Figures 7 and 8.

As shown in Figures 7 and 8, when pretreated with the test compounds, the number of drug injections by pressing the active lever was significantly reduced, indicating the inhibitory effect against cocaine reinstatement.

### Example 4: Experiment about treatment of withdrawal symptoms after drug addiction using animal model

As experimental animals, male Sprague-Dawley rats (260-300 g) were purchased from OrientBio (Seongnam-si, Korea) and provided by the Laboratory Animal Research Center of Sungkyunkwan University. Rats were housed in a breeding room maintained at 22 ± 2°C under a 12-hour light/dark cycle. Each rat was housed alone in a separate cage from acquisition to the end of the experiment and had *ad libitum* access to food and water during the experiment except for the food training period. All experiments were performed during the light cycle between 10:00 and 18:00.

The experiment on the treatment of withdrawal symptoms in morphine addiction was preformed according to previously reported experimental methods (Maldonado et al., 1992) with minor modifications.

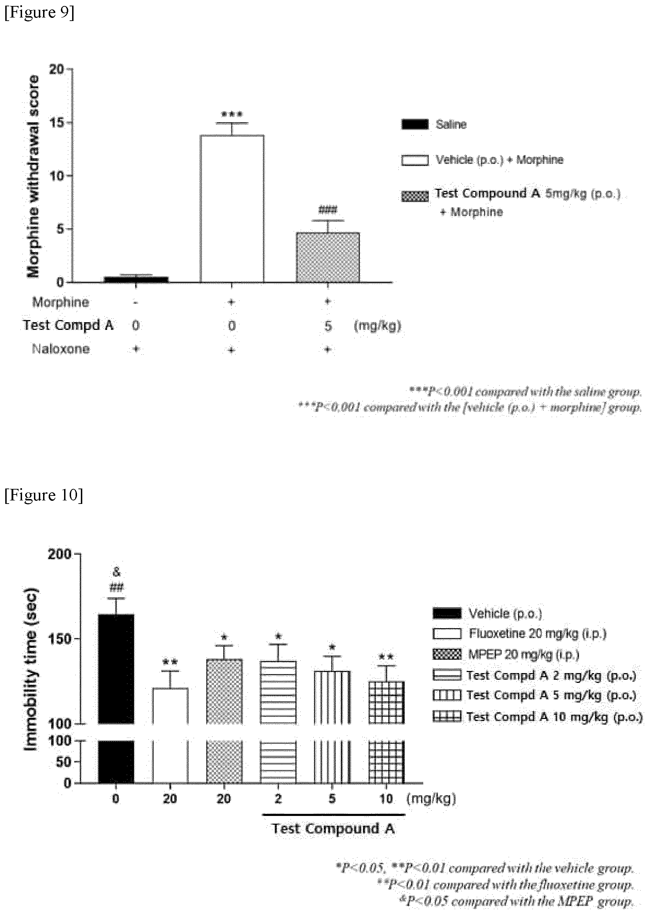

Rats were anesthetized with pentobarbital (50 mg/kg, i.p.) and fixed in a stereotaxic frame (Stoelting Co.). A guide cannula (21 G; Small Parts, Inc., Loganspot, IN, USA) was implanted into the right lateral ventricle of the rat (AP, -0.5 mm; ML, 1.3 mm; DV, 4.5 mm) and then fixed to the skull with dental cement, and the rats were allowed to recover for seven (7) days before osmotic pump implantation. An osmotic pump (Model 2001, Alzet^{™}, Durect Corporation, Cupertino, CA) was filled with saline or morphine (26 nmol/µL) dissolved in saline, and a 3 cm long PE-60 tube (Becton Dickinson, Franklin Lakes, USA) was connected to an injection needle (26 G; Small Parts, Inc., Logansport, IN, USA). The osmotic pump was primed overnight in sterile saline solution at 37°C and then implanted subcutaneously in the dorsal region of the rat. Immediately after transplantation, 1 µL/hour of morphine was infused into the ventricle through the osmotic pump for 72 hours. 72 hours after morphine administration, the tube between the osmotic pump and the injection needle was cut to stop drug administration. 2 hours before the behavioral test, Test Compound A (5 mg/kg) was dissolved in water containing 0.5% HPMC (hydroxypropyl methylcellulose) and 1% Tween 80, and administered orally to the rats. 1 hour before the test, rats were acclimatized in a test box (30 × 30 × 30 cm), and morphine withdrawal syndrome was induced by naloxone administration (2 mg/kg, i.p.) immediately before the behavioral test. Morphine withdrawal syndrome was evaluated for 30 minutes using Maldonado's method, and the results are represented in Figure 9.

**[Table 3] Evaluation method using Maldonado's method**

| Scoring system | | | |
|---|---|---|---|
| Behaviors | 1 | 2 | 3 |
| Rearing | 1-3 | 4-6 | 7< |
| Wet dog shakes | 1-3 | 4-6 | 7< |
| Teeth chattering | 1-3 | 4-6 | 7< |
| Scratching | 1-3 | 4-6 | 7< |
| Grooming | 1-3 | 4-6 | 7< |
| Ptosis (Sec) | 0-200 | 200-400 | 400< |
| Penis-licking | | ○ | |
| Defication | | ○ | |

As shown in Figure 9, it was confirmed that morphine withdrawal symptoms were significantly reduced when pretreated with the test compound, and these results indicate that the test compound is effective in suppressing the withdrawal symptoms of morphine addicts.

### Example 5: Experiment about treatment of depressive disorder using animal model

As experimental animals, 7-8-week-old male BALB/c mice were purchased from OrientBio (Seongnam-si, Korea) and provided by the Experimental Animal Research Center of Naason Science Inc. Mice were housed in a breeding room maintained at a temperature of 22 ± 1°C and humidity of 30-50% under a 12-hour light/dark cycle. During the experiment, mice had *ad libitum* access to food and water.

Test Compound A was dissolved in water containing 0.5% HPMC (hydroxypropyl methylcellulose) and 1% Tween 80, and administered orally at doses of 2, 5 and 10 mg/kg 1 hour before the test. Fluoxetine and MPEP, which are the reference compounds, were dissolved in water for injection and administered intraperitoneally at a dose of 20 mg/kg. The mouse tail suspension test (TST) was performed in a chamber (33 × 33 × 31.75 cm) made of polyvinyl chloride (PVC). A piece of transparent tape was adhered to the tail of each mouse, and the mouse was taped to a transducer that measured tail suspension force. The mouse was videotaped during the 10-minute test period, and the immobility time, which is defined as a state of immobility without agitation, was measured. The results are represented in Figure 10.

As shown in Figure 10, the administration of the test compound resulted in a significant reduction in immobility time compared to the vehicle-administered control group. These results suggest that the test compound is effective as an antidepressant.

### Example 6: Experiment about treatment of anxiety disorder using animal model

As experimental animals, 8-9-week-old male Sprague-Dawley rats were purchased from Koatech (Pyeongtaek-si, Gyeonggi-do, Korea) and provided by the Experimental Animal Research Center of Naason Science Inc. Rats were housed in a breeding room maintained at a temperature of 22 ± 1°C and humidity of 30-50% under a 12-hour light/dark cycle. During the experiment, rats had *ad libitum* access to food and water.

Test compound A was dissolved in water containing 0.5% HPMC (hydroxypropyl methylcellulose) and 1% Tween 80, and administered orally at a dose of 10 mg/kg 2 hours before the test. Diazepam, which is the reference compound, was dissolved in water for injection and administered intraperitoneally at a dose of 1 mg/kg.

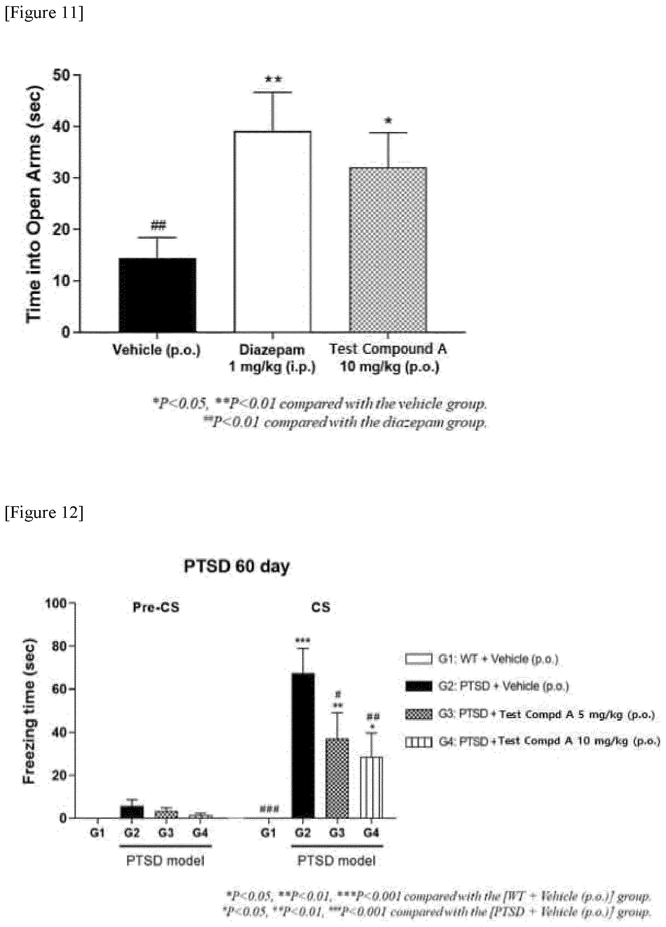

The elevated plus maze (EPM) consisted of black acrylic platforms (diameter: 105 cm, width: 10 cm, height above the ground: 65 cm). The maze was divided into four equal quadrants, two of which were surrounded by 27 cm high black acrylic walls. Walls were placed both inside and outside the platform to provide the "closed" arms of the maze, and the "open" arms have no walls. The camera was placed 2 m above the ground, and the maze was surrounded by a curtain to prevent interference from the surroundings. 2 Hours after drug administration, each rat was placed on the cross maze, and the animal's behavior was recorded for 5 minutes. The results are represented in Figure 11.

As shown in Figure 11, animals that administered the test compound spent significantly more time in the open arms of the maze compared to the vehicle-administered group. These results suggest that the test compound is effective as an anxiolytic.

### Example 7: Experiment about treatment of post-traumatic stress disorder (PTSD) using animal model

As experimental animals, 4-week-old male Sprague-Dawley rats were purchased from Koatech (Pyeongtaek-si, Gyeonggi-do, Korea) and provided by the Experimental Animal Research Center of Naason Science Inc. Rats were housed in a breeding room maintained at a temperature of 22 ± 1°C and humidity of 30-50% under a 12-hour light/dark cycle. During the experiment, rats had *ad libitum* access to food and water.

Post-traumatic stress disorder (PTSD) was induced in rats under the following conditions.
1) Under water trauma (UWT): Rats were acclimatized to the laboratory for 10 minutes before testing and then exposed to swimming for 40 seconds in a water associated zero maze (WAZM) for three (3) consecutive days. On the 4^{th} day of testing, rats were once again exposed to 40 seconds of swimming followed by UWT stress in the WAZM. To provide UWT stress, the rats were placed in the center of the WAZM and then immediately pushed and held in the water using a special metal net (20 × 10 × 15 cm) for 20 seconds, and this was repeated twice. The control group (no PTSD) was exposed to the test for three (3) consecutive days in WAZM without water. On the 10^{th} day of testing, all rats were exposed to WAZM again, and only the PTSD test group was exposed to 40 seconds of swimming.
2) Restrain stress: On the 4^{th} day of testing, after exposure to UWT stress, the animals were restrained in a rat restrainer (IWOO Scientific Corporation, Korea) for 2 hours, and the same stress was applied again on the 10^{th} day of testing.
3) Cued fear conditioning using electric shock: On the 4^{th} day of testing, the rats were acclimatized to a test chamber (Med Associates, USA) for 2 min, and then exposed to a set of 20-second white noise conditioned stimulus (CS) and 1-second electric shock (0.6 mA) a total of 5 times, with a 1-minute interval between each trial. After the last electric shock, the rats were left in the chamber for 30 seconds and then returned to the home cage. Cued fear conditioning was performed again on the 10^{th} day of testing.

Test Compound A was dissolved in water containing 0.5% HPMC (hydroxypropyl methylcellulose) and 1% Tween 80, and administered orally at doses of 5 and 10 mg/kg. Administration of the drug was carried out for five (5) consecutive days for each stage on 11^{th} to 15^{th} days (stage 1), 26^{th} to 30^{th} days (stage 2), and 56^{th} to 60^{th} days (stage 3) of the test. A fear conditioning test was performed 1 hour after the last administration of each stage.

To assess cued fear memory, a contextual and cued fear conditioning test developed for evaluating memory in rats was performed. The rats were returned to the test chamber on 14^{th}, 30^{th}, and 60^{th} days from the beginning of the test. During the cued test, the rats were placed in a novel environment (different wall, new orange scent) for 4 minutes, and the 4-minute exposure consisted of 1 minute of pre-CS (cued stimulus), 2 minutes of CS-test and 1 minute of post-CS. Fear memory was assessed by measuring freezing, defined as complete immobility except for breathing. Freezing behavior was quantified using Actimetrucs Freezeframe software. The quantified test results are represented in Figure 12.

As shown in Figure 12, the animals administered with the test compound exhibited significantly reduced freezing behavior compared to the control test group, and these results suggest that the test compound is an effective therapeutic agent for post-traumatic stress disorder.

## Claims

1. A pharmaceutical composition for the prevention or treatment of mental disorder comprising a compound of the following Formula 1 or a pharmaceutically acceptable salt thereof, together with a pharmaceutically acceptable carrier or excipient: 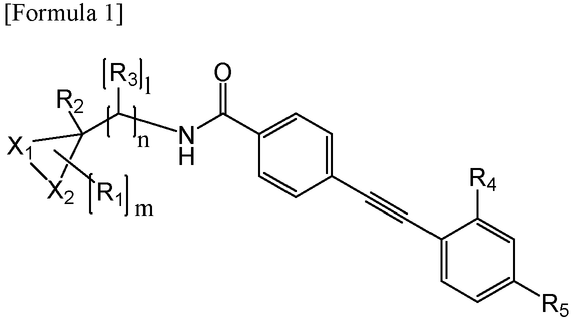 in Formula 1,
X₁ and X₂ together with carbon atom to which they are attached form cyclobutane, cyclopentane, cyclohexane, oxetane, tetrahydrofuran or tetrahydropyran ring;
R₁ is fluoro, hydroxy or C₁-C₅ alkyl;
R₂ is hydrogen, fluoro, hydroxy, C₁-C₅ alkyl or C₁-C₅ alkoxy;
R₃ is hydrogen, deuterium or C₁-C₅ alkyl;
R₄ and R₅ are each independently hydrogen or fluoro, provided that at least one of R₄ and R₅ is fluoro;
l and m are each independently an integer of 0 to 2; and
n is 1 or 2.

2. The pharmaceutical composition for the prevention or treatment of mental disorder according to Claim 1, which comprises a compound of the following Formula 2 or a pharmaceutically acceptable salt thereof, together with a pharmaceutically acceptable carrier or excipient: 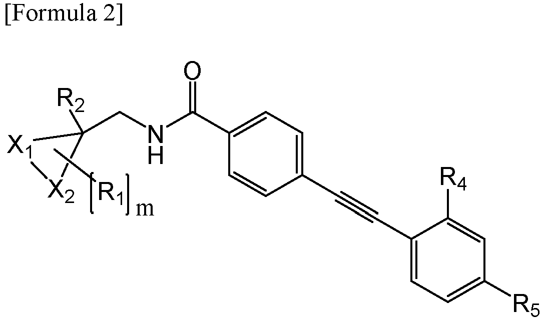 in Formula 2,
X₁ and X₂ together with carbon atom to which they are attached form cyclobutane, cyclopentane, cyclohexane, oxetane, tetrahydrofuran or tetrahydropyran ring;
R₁ is fluoro, hydroxy or methyl;
R₂ is hydrogen, fluoro, hydroxy, methyl, ethyl or methoxy;
R₄ and R₅ are each independently hydrogen or fluoro, provided that at least one of R₄ and R₅ is fluoro; and
m is an integer of 0 to 2.

3. The pharmaceutical composition for the prevention or treatment of mental disorder according to Claim 1, wherein the compound of Formula 1 is selected from the group consisting of:
4-((2-fluorophenyl)ethynyl)-N-((tetrahydro-2H-pyran-4-yl)methyl)benzamide;
N-((4,4-difluorocyclohexyl)methyl)-4-((2-fluorophenyl)ethynyl)benzamide;
4-((2-fluorophenyl)ethynyl)-N-(1-(tetrahydro-2H-pyran-4-yl)ethyl)benzamide;
4-((2-fluorophenyl)ethynyl)-N-((tetrahydrofuran-3-yl)methyl)benzamide;
4-((2,4-difluorophenyl)ethynyl)-N-((tetrahydro-2H-pyran-4-yl)methyl)benzamide;
4-((2-fluorophenyl)ethynyl)-N-((3-methyloxetan-3-yl)methyl)benzamide;
N-((3,3-difluorocyclobutyl)methyl)-4-((2-fluorophenyl)ethynyl)benzamide;
4-((2-fluorophenyl)ethynyl)-N-((4-methoxytetrahydro-2H-pyran-4-yl)methyl)benzamide;
(R)-4-((2-fluorophenyl)ethynyl)-N-((tetrahydrofuran-3-yl)methyl)benzamide;
4-((2-fluorophenyl)ethynyl)-N-((3-hydroxyoxetan-3-yl)methyl)benzamide;
4-((4-fluorophenyl)ethynyl)-N-((3-methyloxetan-3-yl)methyl)benzamide;
4-((2-fluorophenyl)ethynyl)-N-(((1s,3 s)-3 -hydroxy-3 - methylcyclobutyl)methyl)benzamide;
(R)-4-((2-fluorophenyl)ethynyl)-N-((tetrahydro-2H-pyran-3-yl)methyl)benzamide;
(S)-4-((2-fluorophenyl)ethynyl)-N-((tetrahydrofuran-3-yl)methyl)benzamide;
4-((4-fluorophenyl)ethynyl)-N-((tetrahydrofuran-3-yl)methyl)benzamide;
4-((2-fluorophenyl)ethynyl)-N-((3-methyloxetan-3-yl)methyl-d)benzamide;
(S)-4-((4-fluorophenyl)ethynyl)-N-((tetrahydrofuran-3-yl)methyl)benzamide;
(R)-4-((2,4-difluorophenyl)ethynyl)-N-((tetrahydrofuran-3-yl)methyl)benzamide;
(S)-4-((2,4-difluorophenyl)ethynyl)-N-((tetrahydrofuran-3-yl)methyl)benzamide;
(S)-4-((2,4-difluorophenyl)ethynyl)-N-((tetrahydro-2H-pyran-3-yl)methyl)benzamide;
N-((3,3-difluorocyclobutyl)methyl)-4-((2,4-difluorophenyl)ethynyl)benzamide;
4-((2,4-difluorophenyl)ethynyl)-N-((3-ethyloxetan-3-yl)methyl)benzamide;
4-((2,4-difluorophenyl)ethynyl)-N-((3-fluorooxetan-3-yl)methyl)benzamide;
4-((2,4-difluorophenyl)ethynyl)-N-((4-methyltetrahydro-2H-pyran-4-yl)methyl)benzamide;
4-((2,4-difluorophenyl)ethynyl)-N-(2-(tetrahydro-2H-pyran-4-yl)ethyl)benzamide;
4-((4-fluorophenyl)ethynyl)-N-((1-hydroxycyclohexyl)methyl)benzamide;
4-((4-fluorophenyl)ethynyl)-N-((1-hydroxycyclobutyl)methyl)benzamide;
4-((2,4-difluorophenyl)ethynyl)-N-((1-hydroxycyclohexyl)methyl)benzamide;
4-((2,4-difluorophenyl)ethynyl)-N-((1-hydroxycyclobutyl)methyl)benzamide; and
N-(cyclobutylmethyl)-4-((2,4-difluorophenyl)ethynyl)benzamide.

4. The pharmaceutical composition for the prevention or treatment of mental disorder according to Claim 1, wherein the mental disorder is substance-related and addictive disorder.

5. The pharmaceutical composition for the prevention or treatment of mental disorder according to Claim 4, wherein the substance-related and addictive disorder is induced by a substance selected from alcohol; anxiolytics and sedatives (including benzodiazepines, zolpidem, propofol, ketamine, esketamine, phenobarbital); caffeine; cannabis (including marijuana, synthetic cannabis); hallucinogens (including LSD, phencyclidine, psilocybin); inhalants (including paint thinners, some adhesives); opioids (including fentanyl, morphine, oxycodone, pethidine, methadone, hydromorphone, hydrocodone, oxymorphone, codeine, heroin); stimulants (including methamphetamine, ecstasy, amphetamines, cocaine); cigarette; and others (including appetite suppressants such as phentermine, phendimetrazine, diethylpropion and mazindol; synthetic anabolic steroids; other commonly abused substances).

6. The pharmaceutical composition for the prevention or treatment of mental disorder according to Claim 4, which is for treating or attenuating withdrawal symptoms of the substance inducing the substance-related and addictive disorder.

7. The pharmaceutical composition for the prevention or treatment of mental disorder according to Claim 4, which is used in combination with an anti-addiction agent including a drug of substitution or a drug of replacement.

8. The pharmaceutical composition for the prevention or treatment of mental disorder according to Claim 7, wherein the anti-addiction agent is at least one selected from naloxone, naltrexone, nalmefene, disulfiram, acamprosate, topiramate, risperidone, paliperidone, ondansetron, fluoxetine, sertraline, paroxetine, citalopram, fluvoxamine, venlafaxine, duloxetine, mirtazapine and bupropion.

9. The pharmaceutical composition for the prevention or treatment of mental disorder according to Claim 4, which is for preventing recurrence or reducing the likelihood of recurrence of abuse of the substance inducing the substance-related and addictive disorder in a patient attempting to discontinue use of the substance inducing the substance-related and addictive disorder.

10. The pharmaceutical composition for the prevention or treatment of mental disorder according to Claim 1, wherein the mental disorder is depressive disorder or anxiety disorder.

11. The pharmaceutical composition for the prevention or treatment of mental disorder according to Claim 1, wherein the mental disorder is post-traumatic stress disorder.

12. A compound selected from the group consisting of:
4-((2,4-difluorophenyl)ethynyl)-N-(2-(tetrahydro-2H-pyran-4-yl)ethyl)benzamide;
4-((4-fluorophenyl)ethynyl)-N-((1-hydroxycyclohexyl)methyl)benzamide;
4-((4-fluorophenyl)ethynyl)-N-((1-hydroxycyclobutyl)methyl)benzamide;
4-((2,4-difluorophenyl)ethynyl)-N-((1-hydroxycyclohexyl)methyl)benzamide;
4-((2,4-difluorophenyl)ethynyl)-N-((1-hydroxycyclobutyl)methyl)benzamide; and
N-(cyclobutylmethyl)-4-((2,4-difluorophenyl)ethynyl)benzamide,
or a pharmaceutically acceptable salt thereof.
